(19) 

**Europäisches Patentamt**
**European Patent Office**
**Office européen des brevets**

(11) **EP 4 209 175 B1**

(12) **EUROPEAN PATENT SPECIFICATION**

(45) Date of publication and mention
of the grant of the patent:
**01.01.2025  Bulletin 2025/01**

(51) International Patent Classification (IPC):
**A61B 5/296** (2021.01)  **A61B 5/11** (2006.01)
**A41D 1/00** (2018.01)  **A41D 13/00** (2006.01)
**A61B 5/252** (2021.01)  **A61B 5/26** (2021.01)
**A61B 5/251** (2021.01)  **A41D 13/12** (2006.01)
**A61B 5/00** (2006.01)

(21) Application number: **20966644.5**

(22) Date of filing: **25.12.2020**

(52) Cooperative Patent Classification (CPC):
**A41D 13/1281; A41D 1/002; A61B 5/251;**
**A61B 5/252; A61B 5/26; A61B 5/296;**
**A61B 5/6804;** A61B 5/263; A61B 5/27; A61B 5/305;
A61B 2562/0209; A61B 2562/046; A61B 2562/125;
A61B 2562/14

(86) International application number:
**PCT/CN2020/139651**

(87) International publication number:
**WO 2022/134081 (30.06.2022 Gazette 2022/26)**

(54) **DEVICE AND METHOD FOR COLLECTING AND PROCESSING ELECTROMYOGRAPHIC SIGNAL**

VORRICHTUNG UND VERFAHREN ZUM SAMMELN UND VERARBEITEN EINES ELEKTROMYOGRAFISCHEN SIGNALS

SYSTÈME ET PROCÉDÉ DE COLLECTE ET DE TRAITEMENT DE SIGNAL ÉLECTROMYOGRAPHIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**12.07.2023  Bulletin 2023/28**

(73) Proprietor: **Shenzhen Shokz Co., Ltd.**
**Shenzhen, Guangdong 518108 (CN)**

(72) Inventors:
• **ZHOU, Xin**
**Shenzhen, Guangdong 518108 (CN)**
• **SU, Lei**
**Shenzhen, Guangdong 518108 (CN)**
• **LI, Meiqi**
**Shenzhen, Guangdong 518108 (CN)**

• **LIAO, Fengyun**
**Shenzhen, Guangdong 518108 (CN)**
• **QI, Xin**
**Shenzhen, Guangdong 518108 (CN)**

(74) Representative: **Wang, Bo**
**Panovision IP**
**Ebersberger Straße 3**
**85570 Markt Schwaben (DE)**

(56) References cited:
WO-A1-2020/254833    CN-A- 104 720 787
CN-A- 105 232 036    CN-A- 105 748 071
CN-A- 106 821 365    CN-A- 108 553 102
CN-A- 108 670 244    CN-A- 109 998 542
US-A1- 2014 070 949    US-A1- 2014 343 392

## Description

## TECHNICAL FIELD

The present disclosure relates to the field of signal collection and processing, and in particular to a device and method for collecting and processing an electromyography (EMG) signal.

## BACKGROUND

With peoples' attention toward sports scientifics and physical health, intelligent wearable **apparatuses** are developing tremendously. Guidance and monitoring of an intelligent wearable apparatus relies on a sensor and an electrode. At present, the intelligent wearable apparatus is facing a contradiction between comfort and signal quality (stability and signal to noise ratio). Commonly used methods to solve the contradiction include but are not limited to a use of a hydrogel electrode, a skin surface process, a dry electrode material and a gel material fixation, etc. These methods may help improve the quality of a collected signal, but have at least one of the problems of a poor comfort, a short service life, and a complicated operation. However, a direct use of a dry electrode (a metal fabric electrode, a conductive silicon electrode, etc.) may solve the above problems, but an electrode module and a human skin are unlikely to fit with each other, which may lead to an inability to collect the signal and may further increase a noise. In addition, when the human body moves, a contact position between the electrode and the human body may move relative to each other, and the movement of the electrode may result in a failure to collect the signal of a target position, which affects the signal quality and increases the noise, etc. Finally, the dry electrode has a great contact impedance with the human skin, which is more likely to cause a large fluctuation and affect the signal quality.

Documents US 2014/343392 A1 and WO 2020/254833 A1 relate to wearable electromyography devices using electrode modules provided with sucker structures for good skin contact.

Therefore, the present disclosure provides a device for collecting and processing an electromyography (EMG) signal of a human body, which significantly improves the quality of the EMG signal collected by the electrode module at the human body skin under a premise of comfort.

## SUMMARY

The present invention provides a device for collecting and processing an electromyography (EMG) signal of a human body as defined in appended claim 1.

In some embodiments, the at least two electrodes include a first electrode and a second electrode arranged side by side on the surface of the base structure.

In some embodiments, the at least two electrodes further include a reference electrode, and the first electrode, the reference electrode and the second electrode are arranged side by side in sequence on the surface of the base structure.

In some embodiments, the at least two electrodes further include a third electrode and a fourth electrode arranged side by side on the surface of the base structure; and the first electrode is arranged side by side with the third electrode, and the second electrode is arranged side by side with the fourth electrode.

In some embodiments, the electrode module includes a plurality of protruding structures located on surfaces of the at least two electrodes or a surface of the base structure.

In some embodiments, the protruding structures are arranged in an array or randomly distributed on the surfaces of the at least two electrodes or the surface of the base structure.

In some embodiments, the protruding structures are hollow inside, and a hollow part of each of the protruding structures has a filler.

In some embodiments, a height of each of the protruding structures is in a range of 0.5mm~10mm.

In some embodiments, the electrode module may further include a plurality of bump structures located on the surface of the base structure or the surfaces of the at least two electrodes

In some embodiments, heights of the plurality of bump structures are in a range of 10 $\mu$m-80 $\mu$m.

In some embodiments, a distribution density of the plurality of bump structures may be in a range of 2 pieces/2.25mm$^2$~10 pieces/2.25mm$^2$.

In some embodiments, the electrode module further includes a plurality of air holes located on the electrodes and/or the base structure.

The embodiments of the present disclosure provide a wearable apparatus, and the wearable apparatus includes an upper garment and pants, and the upper garment and the pants at least include an EMG signal module for collecting an EMG signal of a human body.

In some embodiments, the upper garment at least comprises: at least one upper garment sensor module configured to collect upper body moving data; at least one upper garment data processing module configured to receive and process the upper body moving data; and an upper garment base configured to carry the at least one upper garment sensor module and the at least one upper garment data processing module.

In some embodiments, the at least one upper garment sensor module at least includes a first upper garment sensor module and a second upper garment sensor module, the first upper garment sensor module is located on a left side of the upper garment base, and the second upper garment sensor module is located on a right side of the upper garment base.

In some embodiments, the first upper garment sensor module and the second upper garment sensor module at least include the EMG signal module, an electrocardiogram (ECG) sensor, a respiration sensor, a temperature sensor, a humidity sensor, an inertial sensor, an acid-base sensor, and an acoustic wave transducer.

In some embodiments, the inertial sensor is located in the upper garment processing module.

In some embodiments, the at least one upper garment data processing module at least includes a first upper garment processing module and a second upper garment processing module; the first upper garment processing module is located at a left shoulder of the upper garment base, and is communicatively connected to the first upper garment sensor module; and the second upper garment processing module is located at a right shoulder of the upper garment base, and is communicatively connected with the second upper garment sensor module.

In some embodiments, the first upper garment processing module and the second garment processing module are in a subordination or in a parallel relationship.

In some embodiments, the pants at least includes: at least one pants sensor module configured to collect lower body moving data; at least one pants data processing module configured to receive and process the lower body moving data; and a pants base configured to carry the at least one pants sensor module and the at least one pants data processing module.

In some embodiments, the at least one pants sensor module at least includes a first pants sensor module and a second pants sensor module, the first pants sensor module is located on a left side of the pants base, and the second pants sensor module is located on a right side of the pants base.

In some embodiments, the first pants sensor module and the second pants sensor module at least include the EMG signal module, an ECG sensor, a respiration sensor, a temperature sensor, a humidity sensor, an inertial sensor, an acid-base sensor, and an acoustic wave transducer.

In some embodiments, the at least one pants data processing module at least includes a first pants processing module and a second pants processing module; the first pants processing module is located on a left side of a left leg of the pants base, and is communicatively connected to the first pants sensor module; and the second pants processing module is located on a right side of a right leg of the pants base, and is communicatively connected to the second pants sensor module.

In some embodiments, the first pants processing module and the second pants processing module are in a subordination or in a parallel relationship.

## BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, wherein:

FIG. 1 is a structural schematic diagram illustrating an electrode module according to some embodiments of the present disclosure;

FIG. 2 is a structural schematic diagram illustrating another electrode module according to some embodiments of the present disclosure;

FIG. 3 is a structural schematic diagram illustrating another electrode module according to some embodiments of the present disclosure;

FIG. 4 is an electrode impedance parallel model according to some embodiments of the present disclosure;

FIG. 5 is an electrode impedance parallel model of a conductive silicon electrode according to some embodiments of the present disclosure;

FIG. 6 is a diagram illustrating a relationship between a conductive silicon electrode and its thickness according to some embodiments of the present disclosure;

FIG. 7A is a structural schematic diagram illustrating an electrode according to some embodiments of the present disclosure;

FIG. 7B is a parallel model of a metal fabric conductive silicon composite electrode according to some embodiments of the present disclosure;

FIG. 8 is a structural schematic diagram illustrating a sucker structure according to some embodiments of the present disclosure;

FIG. 9 is a cross-sectional diagram illustrating another sucker structure according to some embodiments of the present disclosure;

FIG. 10 is a cross-sectional diagram illustrating another sucker structure according to some embodiments of the present disclosure;

FIG. 11 is a cross-sectional diagram illustrating another sucker structure according to some embodiments of the present disclosure;

FIG. 12 is a structural schematic diagram illustrating an electrode module with a sucker structure according to some embodiments of the present disclosure;

FIG. 13 is a structural schematic diagram illustrating another electrode module with a sucker structure according to some embodiments of the present disclosure;

FIG. 14 is a structural schematic diagram illustrating an exemplary wearable apparatus according to some embodiments of the present disclosure;

FIG. 15 is a structural diagram illustrating an exemplary wiring connection mode of an upper garment wearable apparatus according to some embodiments of the present disclosure;

FIG. 16A is a structural diagram illustrating an exemplary elastic design at an underarm position of an upper garment wearable apparatus according to some embodiments of the present disclosure;
FIG. 16B is a structural diagram illustrating an exemplary elastic design at an upper arm position of a wearable apparatus according to some embodiments of the present disclosure;
FIG. 17A is a structural diagram illustrating an exemplary annular elastic design of an electrode module of a wearable apparatus according to some embodiments of the present disclosure;
FIG. 17B is a structural diagram illustrating an exemplary direction-specific elastic design of an electrode module of a wearable apparatus according to some embodiments of the present disclosure;
FIG. 18 is a structural diagram illustrating an exemplary hollow-carved design of a wearable apparatus according to some embodiments of the present disclosure;
FIG. 19A is a structural diagram illustrating an exemplary knife-shaped electrode module according to some embodiments of the present disclosure; and
FIG. 19B is a cross-sectional view of an exemplary knife-shaped electrode module according to some embodiments of the present disclosure.

## DETAILED DESCRIPTION

In order to more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.
It should be understood that "system", "device", "unit" and/or "module" as used herein is a mode for distinguishing different components, elements, parts, or assemblies of different levels. However, if other words may achieve the same purpose, the words may be replaced by other expressions.
As used in the disclosure and the appended claims, the singular forms "a," "an," and "the" include plural referents unless the content clearly dictates otherwise; the plural forms may be intended to include singular forms as well. In general, the terms "comprise," "comprises," and/or "comprising," "include," "includes," and/or "including," merely prompt to include operations and elements that have been clearly identified, and these operations and elements do not constitute an exclusive listing. The methods or devices may also include other operations or elements.
Flowcharts are used in this description to illustrate the operation implemented according to the system of the embodiments of the present disclosure. It should be understood that the previous or subsequent operations may not be accurately implemented in order. Conversely, the operations may be implemented in inverted order, or simultaneously. Meanwhile, other operations may further be added to these processes, or a certain operation or several operations may be removed from these processes.
With people's attention toward sports scientifics and physical health, an intelligent wearable apparatus is developing tremendously, and the intelligent wearable apparatus mainly relies on an electrode to collect a bioelectrical signal. Specifically, as a kind of the bioelectrical signal, an electromyography (EMG) signal is a source of the electrical signal that generates a muscle power. It is a superposition of action potentials of many motor units in the muscle in time and space, which largely reflects a moving state of a neural and a muscle. There are generally two methods for collecting the EMG signal. One is to collect the EMG signal by inserting a needle electrode into the muscle, that is, a needle EMG signal. It has advantages of little interference and easily identifiable, but it will cause harm to a human body. Another method is to collect the EMG signal on a human skin surface by sticking an electrode slice on the human skin, that is, a surface EMG (SEMG) signal. This method is generally used to collecting the SEMG signal when a human body is still. When the human body is moving, there is a relative movement between the electrode and the human body, making the collected EMG signal inaccurate. At present, commonly used methods include but are not limited to a use of a hydrogel electrode, a skin surface process, a dry electrode material and a gel material fixation, etc. These methods may help improve the quality of a collected signal, but have at least one of the problems of a poor comfort, a short service life, and a complicated operation.
An electrode module and the intelligent wearable apparatus (such as clothing, a wristband, a shoulder strap, etc.) described in the embodiments of the present disclosure mainly involves the collection of a skin surface EMG signal (hereinafter collectively referred to as an EMG signal). The EMG signal may be obtained from many parts of the human body, such as calves, thighs, a hip, a waist, a back, a chest, shoulders, a neck, etc., and the EMG signal obtained from different parts carries movement and function information of the corresponding parts. For example, the EMG signal on the leg reflects a posture and moving state of the leg, such as walking, running, squatting, etc. Therefore, it is possible to collect the EMG signal through the intelligent wearable apparatus to meet people's requirement for exercise and fitness guidance. To collect the EMG signal of a user's body from many aspects when the user is exercising, the intelligent wearable apparatus may be the clothing. Through setting electrode modules corresponding to various parts of the human body (e.g., the calves, the thighs, the hip, the waist, the back, the chest, the shoulders, and the neck, etc.), the EMG signals of the various parts of the user's body when exercising may be collected. Optionally, the intelligent wearable apparatus may directly analyze and process the collected EMG signals, or transmit the collected EMG signals to a processing terminal (e.g., a mobile terminal device, a cloud server) through wired or wireless means for analyzing and processing to determine whether the user's movement is correct, and then provide corresponding feedback to the user to correct the user's incorrect movement. Further, the intelligent wearable apparatus may further formulate a scientific personal exercise plan for the user based on the collected EMG signals, and guide the user to exercise. In addition, a very important value lies in applying the EMG signal to a study of a human moving state. For example, in a medical rehabilitation engineering, the EMG signal may be used for patient treatment. In bionics, the EMG signal may be used for research on artificial limbs.

When the clothing is used as an intelligent wearable apparatus to collect the EMG signal when the user exercises, the electrode module may not only collect the EMG signal with a better quality, but also have features including a softness, an ultra-thinness, a tolerance, a skin-friendly, etc., so as to avoid a discomfort brought by the electrode to the user when the user wears the clothing for exercise and fitness.

In some embodiments, an amplitude of an original EMG signal directly collected by the electrode is very small, and has a great noise, so processes including a filtering and a differential amplification, etc. are required before the subsequent analysis or processing on the EMG signal are performed.

In some embodiments, the noise in the original EMG signal mainly includes a common-mode (CM) noise, and the CM noise is mainly caused by a power-frequency CM signal. Therefore, to reduce the CM noise, the power-frequency CM signal may be effectively restrained through a differential circuit, a right leg drive circuit, a filter circuit, etc., thereby reducing or eliminating the CM noise. According to preference for example, the differential circuit and the right leg drive circuit may be used to effectively restrain the power frequency CM signal. In some embodiments, considering that the CM noise is mainly caused by the power frequency, the frequency of the CM noise is mainly concentrated around 50 Hz or 60 Hz (or its harmonics), and a frequency distribution of a main intensity of the EMG signal is 20~140 Hz, so the CM noise may be filtered out by one or more filters or notch filters. For example, in some embodiments, a 50 Hz notch filter and a 140 Hz low pass filter may be used to filter out the CM noise. For another example, a 50 Hz notch filter, a 100 Hz notch filter, a 150 Hz notch filter, and a 240 Hz low pass filter may be used to filter out the CM noise. For another example, a 50 Hz notch filter, a 150 Hz notch filter, and a 230 Hz low-pass filter may be used to filter out the CM noise.

In some embodiments, the noise in the original EMG signal may also include a differential mode noise caused by a conversion of the CM signal into a differential mode signal. For example, when a differential circuit is used to restrain the CM noise, a fluctuation of a contact impedance between the electrodes and the skin may cause an inconsistency in the contact impedance between the two electrodes corresponding to the differential circuit and the human skin, thus making the CM signal converse into the differential signal and causing the differential mode noise. Especially, a differential amplifier circuit may amplify the differential mode signal. Therefore, an amount of conversion of the CM signal to the differential mode signal may be reduced by reducing or avoiding the contact impedance fluctuation. For example, a value of the contact impedance between the electrodes and the skin may be reduced so that the contact impedance has a smaller fluctuation range, thereby reducing the differential mode noise caused by the fluctuation of the contact impedance.

In some embodiments, the contact impedance the two electrodes corresponding to the differential circuit being inconsistent with the contact impedance of the skin caused by the contact impedance fluctuation may further cause a voltage division problem, so that an amplitude of the collected EMG signal may be small and does not have a sufficient strength. Therefore, the problem of voltage division can be solved by increasing the input impedance of the circuit connected to the electrodes (e.g., a differential circuit). When the difference between the input impedance and the contact impedance is large enough, the impact of the contact impedance fluctuation on the strength of the EMG signal can be reduced or avoided.

In some embodiments, the noise may be reduced by reducing the contact impedance, so as to improve a quality of the EMG signal. In some embodiments, the quality of the EMG signal may further be graded, so as to remove a poor-quality EMG signal and keep the high-quality EMG signal, or use an algorithm to process the poor-quality EMG signal into the high-quality EMG signal. Specifically, a contact impedance evaluation circuit may be designed to connect to the electrode, and the contact impedance evaluation circuit may be used to measure a size or an abnormality of the electrode contact impedance, which may directly affect the quality of the collected EMG signal. Therefore, a coefficient used to measure the contact impedance may be taken as an indicator of the quality of the collected EMG signal, and the indicator may be used to assign a weight to the EMG signal collected. The greater the weight is, the higher the quality of the EMG signal is.

In some embodiments, to more accurately measure the EMG signals of various parts of the human body, when collecting the EMG signals of the various parts of the human body, different numbers of electrodes may be arranged correspondingly in the various parts of the human body. For example, a plurality of electrodes for collecting the EMG signals of a certain part may be integrated into an electrode module, and the electrode module may be arranged at a muscle center area of the part and may include at least two electrodes, and the at least two electrodes may be arranged along a length direction of muscle fibers of the part in sequence

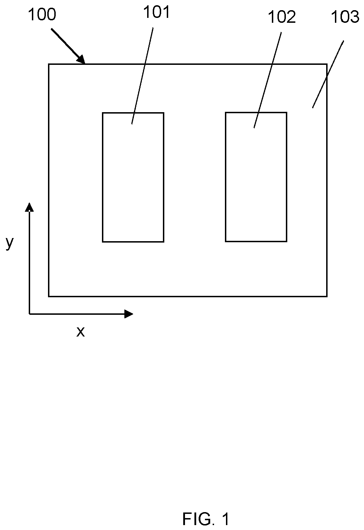

FIG. 1 is a structural schematic diagram illustrating an electrode module according to some embodiments of the present disclosure. In some embodiments, as shown in FIG. 1, an electrode module 100 may include a base structure 103 and two electrodes (also referred to as electrode units). The two electrodes are spaced apart on a surface of the base structure 103. In some embodiments, the base structure 103 may be made of a flexible insulating material (e.g., a resin, a soft PVC, a silicone), and a shape of the base structure may be a rectangle, a circle, or other irregular shapes. The electrodes may be fixedly connected to the base structure 103 by pasting, clamping, welding, etc., and the electrode module 100 may be fixed at a corresponding position of a wearable apparatus through the base structure 103. Specifically, in some embodiments, the two electrodes may include a first electrode 101 and a second electrode 102. When the electrode module 100 is used to collect the EMG signal, the first electrode 101 and the second electrode 102 may be arranged along a length direction of muscle fibers (x direction in FIG. 1) in sequence. Different positions along the length direction of the muscle fiber have different potentials. The position of the muscle fiber where the first electrode 101 is located has a first potential, and the position of the muscle fiber where the second electrode 102 is located has a second potential. There is a potential difference between the first potential and the second potential (the potential difference may be used to reflect the EMG signal). In some embodiments, the electrode module may further include a differential circuit, and the first electrode and the second electrode may be electrically connected to two input ends of the differential circuit respectively. The differential circuit may refer to a circuit structure that amplifies a differential mode signal and restrains a CM signal.

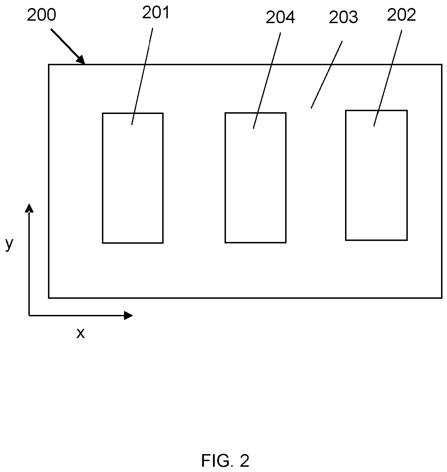

FIG. 2 is a structural schematic diagram illustrating another electrode module according to some embodiments of the present disclosure. In some embodiments, as shown in FIG. 2, an electrode module 200 may include a first electrode 201, a third electrode 204, and a second electrode 202 arranged at intervals along a length direction of a muscle fiber on a surface of a base structure 203. A position of the muscle fiber where the first electrode 201 is located has a first potential, a position of the muscle fiber where the second electrode 202 is located has a second potential, and a position of the muscle fiber where the third electrode 204 is located has a third potential. Further, there is a potential difference between the first potential and the second potential, the first electrode 201 and the second electrode 202 are respectively connected to input ends of a differential circuit. The third electrode 204 in the middle may be taken as a reference electrode for recording motion artifacts (MA) noise in the EMG signal collected by the electrode module and a noise caused by a poor contact, and then the noises may be weakened to a certain extent. The MA refers to the noise caused by a movement, such as a change of an electrode fit or a position caused by the movement, which increases the noise in the output signal. In some embodiments, an MA noise frequency may be related to the motion, and the MA noise may be filtered out by a high pass filter. However, in some cases, the MA noise may have a multiplicative effect with other sources, resulting in a superposition of the frequencies. At this time, it is difficult to filter out the MA noise through the filter. To better deal with the problem of the MA noise, in some embodiments, the third electrode 204 may be provided between the first electrode 201 and the second electrode 202. First, the third electrode 204 is located between the first electrode 201 and the second electrode 202. The third electrode 204 has a reference meaning for the electrode module 200. The third electrode 204 may be measured separately to obtain its relevant information, such as a contact impedance, a power frequency output, etc. The relevant information of the third electrode 204 may be introduced into an evaluation system and taken as a supervision value for determining a quality of the EMG signal collected by the electrode module 200. Second, the third electrode 204 is located between the first electrode 201 and the second electrode 202, and the third electrode 204 may change with a change of a state of the entire electrode module 200. For example, the MA noise introduced when the first electrode 201 and the second electrode 202 are measuring the EMG signal or other noises caused by a non-attachment of the electrodes to the skin may further be reflected on the third electrode 204. As an example only, when the electrode module 200 only includes the first electrode 201 and the second electrode 202, a reference point (where potential is 0, e.g., GND) of a measurement circuit of the electrode module 200 is connected to the first electrode 201, the second electrode 202, or a fixed source, and a measurement result of the electrode module is actually a result of a differential amplification for a difference between the first electrode 201 and the second electrode 202. When the electrode module 200 is provided with the third electrode 204, the reference point is connected to the third electrode 204, and the measurement result of the electrode module is a result of a differential amplification for a difference between a potential difference between the first electrode 201 and the third electrode 204 as well as a potential difference between the second electrode 202 and the third electrode 204. The potential obtained by measuring the third electrode 204 contains the noise caused by the MA and the poor contact, etc. By measuring the potential difference between the first electrode 201 and the third electrode 204 and the potential difference between the second electrode 202 and the third electrode 204, the noise caused by the MA and the poor contact may be effectively reduced. Third, the third electrode 204 may be taken as an output end of a right leg drive circuit. The right leg drive circuit collects a CM signal of the electrode module, amplifies the direction, and then feedback the CM signal to the human body, which effectively restrains the CM signal from the source. In addition, the third electrode 204 may be used as an EMG signal collection electrode similar to the first electrode 201 and the second electrode 202, which helps to implement a collection combination among different electrode pairs. For example, the first electrode 201 and the third electrode 204 may collect a group of EMG signals, and the second electrode 202 and the third electrode 204 may collect another group of EMG signals. Through properties of these two groups of EMG signals (e.g., within a certain range, the strengths of the EMG signals are positively related to distances between the electrode collection), the EMG signals may be extracted to optimize a signal-to-noise ratio (SNR). In some embodiments, the base structure 203 is the same as or similar to the base structure 103.

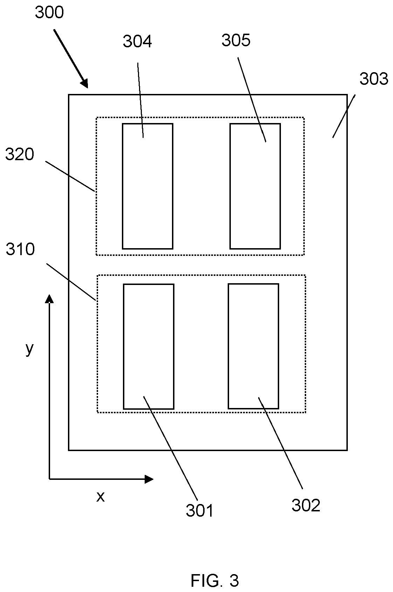

FIG. 3 is a structural schematic diagram illustrating another electrode module according to some embodiments of the present disclosure. In some embodiments, as shown in FIG. 3, an electrode module 300 may include two groups of electrodes, and the two groups of electrodes are arranged at intervals along a width direction (y direction) of a muscle fiber on a surface of a base structure 303. The two electrodes of each group of electrodes are arranged at intervals along a length direction of the muscle fiber. Specifically, the two groups of electrodes are respectively a first group of electrodes 310 and a second group of electrodes 320, the first group of electrodes 310 includes a first electrode 301 and a second electrode 302, and the second group of electrodes 320 includes a third electrode 304 and a fourth electrode 305. A position of the muscle fiber where the first electrode 301 is located has a first potential, the position of the muscle fiber where the second electrode 302 is located has a second potential, the position of the muscle fiber where the third electrode 304 is located has a third potential, and the position of the muscle fiber where the fourth electrode 305 is located has a fourth potential. In some embodiments, the first electrode 301 and the third electrode 304 are jointly connected to an input end of a differential circuit, and the greater value, smaller value or an average value of the first potential and the third potential may be taken as an input of the input end. The second electrode 302 and the fourth electrode 302 are connected to the other input end of the differential circuit. The greater value, smaller value or an average value of the second potential and the fourth potential may be taken as the input of the other input end of the differential circuit. An EMG signal at the position of the electrode module 300 may be obtained based on the inputs of the two input ends of the differential circuit. In some other embodiments, the first electrode 301 and the second electrode 302 may be respectively connected to two input ends of the differential circuit, and the third electrode 304 and fourth electrode 305 may be respectively connected to two input ends of another differential circuit. At this time, there is a first potential difference between the first potential and the second potential (that is, a first differential signal or a first EMG signal), and there is a second potential difference between the third potential and the fourth potential (that is, a second differential signal or a second EMG signal). Based on the first EMG signal and the second EMG signal, EMG signal crosstalk effect information near the muscle fiber in the Y direction may be obtained, and based on the EMG signal crosstalk effect information, an EMG signal interference of the muscle near the part to be collected may be eliminated through an algorithm, thereby collecting a more accurate EMG signal. In this embodiment, a combination of EMG signals from a plurality of positions of the human body parts corresponding to the electrode module may be extracted through two groups of electrodes, so as to extract more accurate EMG signals.

It should be noted that the above embodiments are only for illustration, and a number and distribution of the electrodes in the electrode module are not limited to those shown in FIG. 1, FIG. 2 and FIG. 3, as long as at least two of the plurality of electrodes in the electrode module are arranged at intervals along the length direction of the muscle fiber, which is not repeated here.

When at least two electrodes (e.g., the first electrode and the second electrode) of the electrode module are fitted to the skin to collect the EMG signal, a contact impedance may form when each electrode contacts the skin. There may be certain changes in a pressure, a position, etc. between the electrodes and the skin, which cause a fluctuation on the contact impedance, thereby affecting the accuracy of the EMG signals. In some embodiments, to reduce a differential mode signal noise caused by a great difference in contact impedance formed when the at least two electrodes (e.g., the first electrode and the second electrode) in the electrode module are fitted to the human skin, the contact impedance fluctuation may be reduced. Specifically, the contact impedance between the at least two electrodes and the skin may be reduced so that the contact impedance does not fluctuate in a greater range, and the difference of the contact impedance at both ends of the electrode may be small, or the contact impedance of the two ends of the electrode may be consistent with each other.

For the convenience of understanding and explanation, when the electrode is fitted to the skin for an EMG signal collection, the contact between the electrode and the skin may be equivalent to a parallel connection of a capacitor and a resistor, and the contact impedance may be calculated by a formula (1):

$$|z| = \frac{d\rho}{S \cdot \sqrt{1 + \omega^2 \varepsilon^2 \rho^2}} \ , \ (1)$$

where, |z| indicates the contact impedance, S indicates a contact area between the electrode and the skin, d indicates a distance between the electrode and the skin, $\rho$ and $\varepsilon$ indicate an average resistivity and a capacitance dielectric coefficient between the electrode and the skin, respectively, $\omega=2\pi\cdot f$, f indicates an EMG signal frequency. From the formula (1), it may be concluded that the greater the electrode area is and the smaller the distance between the electrode and the skin is, the contact impedance between the electrode and the skin is.

Further, to make the contact impedance between the electrode and the skin smaller, the contact area between the electrode and the skin may be increased as much as possible, so a shape and a size of the surface where the electrode contacts the skin may be designed to ensure that the contact area between the electrode and the skin is greater. In some embodiments, the surface of the electrode in contact with the skin may be rectangular, and a length of the surface of the electrode in contact with the skin may be 1 cm to 10 cm, and a width may be 1 cm to 4 cm. For example, the size of the surface may be 4cm×2cm, 3cm×1.5cm, or 2cm×1cm. In some embodiments, the shape of the electrode may further be other regular or irregular shapes such as a circle, a triangle, a hexagon, etc. In a practical application, the shape of the electrode may depend on a muscle shape of the part where the EMG signal is to be collected.

It should be noted that the size of the surface of the electrode in contact with the skin is not limited to the above sizes, and may be greater or smaller, which may be determined according to the part measured. For example, the size and distribution of the electrode may be limited by muscle size and a crosstalk between the muscles. In some embodiments, the electrodes may be distributed in a center of the muscle to be measured but not beyond the range of the muscle. In some embodiments, when the electrodes is in a densely muscled area, the electrode may be located at the center of a target muscle and away from other muscles other than the target muscle. As an example only, when collecting the EMG signal from the muscles of different parts of the human body, the electrodes with different lengths may be selected according to the lengths of the muscle fibers in the length direction. For example, for upper body muscles, the electrodes with a length of 4 cm may be used to collect the EMG signal. For another example, for a part of the muscle with a large range, such as a latissimus dorsi muscle, the electrodes with a length of 10 cm may be used for the collection. For another example, when there is a reference electrode in the electrode module (e.g., the third electrode 204 in FIG. 2), the reference electrode may be smaller than the electrode used to collect the EMG signal (e.g., the first electrode 201 and the second electrode 202 in FIG. 2) in length. For example, the latissimus dorsi muscle may be collected with an electrode with a length of 10 cm, and the length of the reference electrode may be 6 cm. For another example, for a part of the muscle with a small range, the electrode may use a relatively small sized electrode, such as 2 cm or 3 cm. It may be understood that the length of the electrode refers to the length of the electrode along the direction perpendicular to the length of the muscle fiber (the y direction in FIG. 1).

In some embodiments, for the electrode module including at least two electrodes, the distance between two adjacent electrodes may affect an intensity of the measured EMG signal. Taking the electrode module including two electrodes (e.g., the first electrode 101 and the second electrode 102) shown in FIG. 1 as an example, the intensity of the EMG signal may be reflected by the potential difference between the two electrodes. The two electrodes are arranged at intervals along the direction of the muscle fiber (the x direction in FIG. 1). When the user is moving, the muscle to be measured expands and contracts, and there is a potential difference along the direction of the muscle fiber. The two electrodes are arranged at intervals along the length direction of the muscle fiber to collect the potential difference of the muscle to be measured when the human body moves. When the distance between the two electrodes is too small, the intensity of the EMG signal may be affected, and if the distance is too great, the electrodes may easily exceed the muscle range. In some embodiments, to improve the accuracy of the EMG signal, the distance between the two electrodes in the electrode module (the first electrode 101 and the second electrode 102) may be 1 mm~10 cm. According to preference for example, the distance between the two electrodes in the electrode module may be 0.5cm~6cm, 0.8 cm~4 cm, or 1cm~3cm. For different muscle areas, the distance between the two electrodes corresponding to each muscle area may be the same as or different from the distance between the two electrodes corresponding to other muscle areas.

Combined with FIG. 1, the distance between the electrodes of the electrode module and the width of the electrodes (i.e., electrode width) in the muscle fiber direction (i.e., the x direction), may be limited by an EMG signal frequency collection range and an EMG signal conduction speed. Specifically, in an actual measurement, each muscle movement of different individuals corresponds to different EMG signal frequency domains, and different EMG signal frequency domains correspond to different EMG signal frequency collection ranges. In some embodiments, the EMG signal frequency collection range is 10 Hz-200 Hz. When the distance between the electrodes does not correspond to the EMG signal frequency collection range and the EMG signal conduction speed, a transmission of the EMG signal on a body surface may cause a signal frequency domain distortion. For example, when the distance between two electrodes receiving the EMG signal is 1cm, it can be assumed that one of the two electrodes has a point a, the other electrode has a point b, the distance between the point a and the point b is 1cm, and the transmission speed of the EMG signal is 4m/s, then a 400 Hz signal transmitted from the point a may be propagated to the point b and be in phase with the 400 Hz signal at point a, then this 400 Hz signal may appear in phase at the two input ends of the differential circuit and be restrained by a CM rejection in the circuit. As a result, the signal intensity at this frequency decreases, and the time domain signal is distorted.

Combined with an actual EMG signal frequency collection range, in some embodiments, the center-to-center distance between the two electrodes may be less than 3 cm, so that the EMG signal within the EMG signal collection frequency range (e.g., within 133 Hz) may be fully reserved. In some embodiments, a greater center-to-center distance may be adopted. For example, when the center-to-center distance between two electrodes is 6 cm, more EMG signals within the EMG signal collection frequency range (e.g., within 66.5 Hz) may be reserved. In the embodiment of the present disclosure, the center-to-center distance between the two electrodes is a distance between the centers of the two electrodes, which may be expressed as a sum of one half of the width of one electrode, one half of the width of the other electrode, and the distance between adjacent edges of the two electrodes at the x direction. In some embodiments, the electrode width may range from 1 mm to 5 cm. According to preference for example,, the electrode width may range from 0.5 cm to 3 cm. According to preference for example,, the electrode width may be 1 cm, 1.5 cm or 2 cm.

When an electrode has a greater size, the electrode has a greater contact area with the skin, so that the contact impedance between the electrode and the skin may be effectively reduced. Further, when the electrode has a greater size, the electrode may not completely fall off the skin under various movements or wrinkles, so as to collect the EMG signal, and by the collected EMG signal, the effective EMG signal may be extracted through a back-end circuit and an algorithm, etc. In addition, when the electrode with a greater size and the electrode with a smaller size have the same wrinkle, the effect of the wrinkle on the electrode with a larger size may be relatively smaller.

In some embodiments, the contact impedance between the electrodes and the skin as well as the total impedance of the electrodes may be effectively reduced by using electrodes of appropriate materials and structures. In some embodiments, the electrodes may be composed of a single material, such as a metal fabric electrode, a conductive silicon electrode, a hydrogel electrode, a metal electrode, etc. In some embodiments, when the electrodes are used to be integrated on a clothing to fit the skin, according to preference for example,, the electrodes may be composed of the metal fabric and the conductive silicon. According to preference for example,, the electrode may be a metal fabric electrode, as the metal fabric electrode has a smaller resistivity, and its impedance and contact impedance with the skin are also smaller. The smaller a thickness of the metal fabric electrode is, the smaller its impedance and the contact impedance with the skin are. In some embodiments, when a metal fabric electrode is used to collect the EMG signal, according to preference for example, the thickness of the metal fabric electrode may be 10 $\mu$m~5 mm., 100 $\mu$m~3 mm, or 500 $\mu$m~2 mm. In some embodiments, the electrodes may further be formed by stacking different materials, such as a metal fabric conductive silicon composite electrode (also referred to as composite electrode in brief) made of the metal fabric and the conductive silicon, which has a small contact impedance with the skin. Moreover, the conductive silicon in the material contacting the skin is skin-friendly and washable, so as to avoid the discomfort brought by the contact between the electrode and the skin.

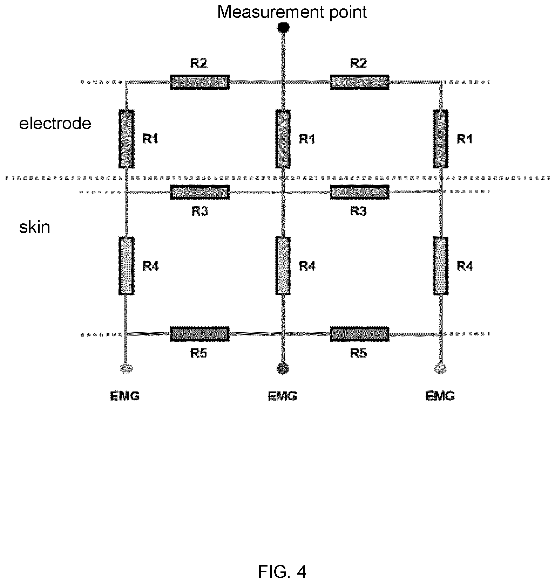

In practical applications, the one end of the electrode connecting the skin may be regarded as a face, while the other end is connected to the wire and may only be regarded as a point. But in specific cases (e.g., when the electrode material has a good conductivity), the end connecting the wire may still be regarded as a face. Therefore, the contact between the electrode and the skin may generally be equivalent to an electrode impedance parallel model shown in FIG. 4. When the electrode material has a good conductivity, R1=0 and R2=0 in FIG. 4.

In FIG. 4, R1 indicates an electrode material longitudinal impedance, R2 indicates an electrode material transverse impedance, R3 indicates a skin transverse impedance, R4 indicates a skin longitudinal impedance (for the convenience of explanation and illustration, the skin longitudinal impedance here includes the contact impedance), R5 indicates a dermis transverse impedance, the EMG signal indicates the EMG signal source, and a measurement point is the point where a conductive wire on the electrode leads out. The R1 and the R2 are related to the material (resistivity), the structure and the size (the direction perpendicular to the skin surface is the thickness, and the direction parallel to the skin surface is the length) of the electrode. As the resistivity of the human skin is a physiological feature, the R3 and the R4 may be a fixed value, but R5 is further affected by the length of the electrode. Therefore, the electrode impedance parallel model may be optimized by changing the electrode material, structure and size (e.g., by changing the R1 and the R2, a parallel path in the electrode impedance parallel model may be changed, thereby reducing the total electrode impedance and the contact impedance), so that an appropriate material may be selected for the electrode and an appropriate structure may be designed.

In some embodiments, the skin surface is a stratum corneum of the human skin, and the stratum corneum is relatively dry and has a relatively high resistivity. Therefore, the R3 has a relatively great value, so it may be regarded as an open circuit. Apart from that, because of a symmetry, conditions of the signal sources are basically the same, so only one EMG signal source may be considered.

In some embodiments, the electrode parallel impedance models corresponding to electrodes of different materials are different, and the parallel paths in the corresponding electrode impedance parallel models may be changed by changing the structure of the electrodes, so as to reduce a total electrode impedance and the contact impedance between the electrodes and the skin.

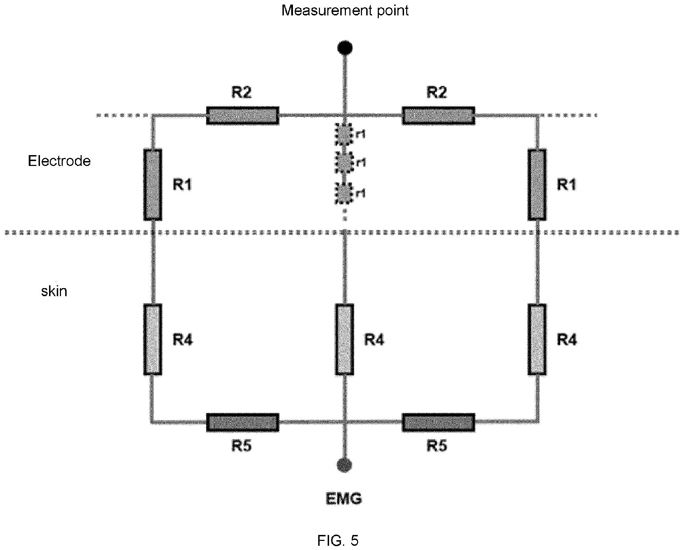

For example, FIG. 5 is an electrode impedance parallel model of a conductive silicon electrode according to some embodiments of the present disclosure. Due to a high resistivity of the conductive silicon, the R1 and the R2 may not be ignored, and the R1 and R2 of the electrodes with different structures and sizes may further vary greatly. The resistivity may have the same effect on the R1 and the R2, while the thickness may have different effects on the R1 and the R2. The greater the thickness is, the smaller the R2 is, so the R2 may be regarded as a parallel connection of a series of transverse impedances r2 made of materials of unit thickness. As shown in FIG. 5, the R1 is further affected by the thickness, the greater the thickness is, the greater the R1 is, so the R1 may be regarded as the parallel connection of a series of transverse impedances r1 made of materials of unit thickness

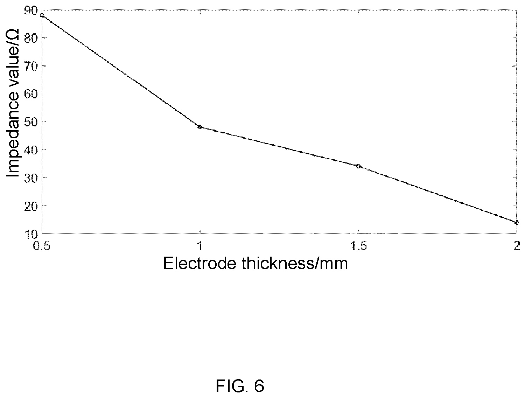

The relationship between the impedance value of the conductive silicon electrode and its thickness in FIG. 6 may be obtained through the electrode impedance parallel model of the conductive silicon electrode shown in FIG. 5. It can be seen from FIG. 6 that the greater the thickness of the conductive silicon electrode is, the smaller its impedance is. For the electrode made of the material with greater resistivity (such as the conductive silicon electrode), the thickness of the electrode may be increased to reduce the transverse resistance R2 of the electrode, so that a path with a greater area may join the parallel model (e.g., assuming that an impedance value of the parallel path is equal to or more than ten times the minimum resistance path, which may be ignored, and the smaller electrode transverse resistance R2 may bring a path with a greater area to join the parallel connection), and the parallel connection may greatly reduce the total impedance and the contact impedance. In some embodiments, the conductive silicon electrode may have a thickness of 0.01 mm-4mm. According to preference for example, the thickness of the conductive silicon electrode may be 0.1mm-3mm, or 0.5mm-1mm. In some embodiments, the conductive silicon electrodes may have a width of 1 mm to 5 cm. According to preference for example, the width of the conductive silicon electrode may be 0.5cm-3cm. According to preference for example, the width of the conductive silicon electrode may be 1 cm, 1.5 cm or 2 cm. In some embodiments, the length of the surface of the conductive silicon electrode contacting the skin may be 1 cm to 10 cm. It should be noted that the length of the conductive silicon electrode may be determined according to a part measured. For example, for an upper body muscle, a conductive silicon electrode with a length of 4 cm may be used to collect the EMG signal. For another example, for parts of the muscle with a large range, such as the latissimus dorsi, a conductive silicon electrode with a length of 10 cm may be used to collect the EMG signal.

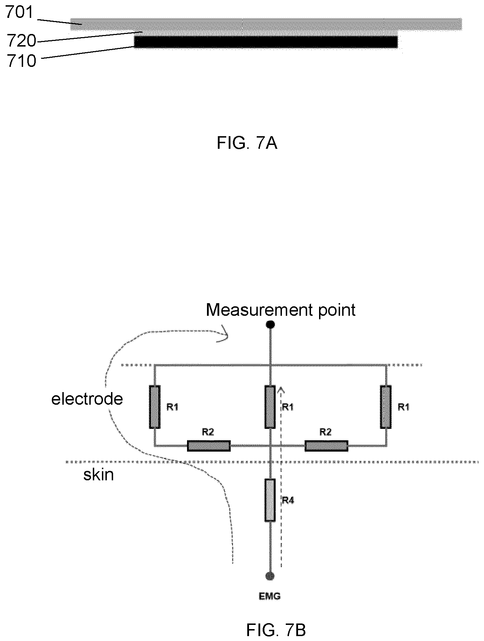

In some embodiments, electrodes of different materials may be combined to reduce the total impedance of the electrodes. As an illustration only, the metal fabric conductive silicon composite electrode is taken as an example for description. FIG. 7A is a structural schematic diagram illustrating an electrode according to some embodiments of the present disclosure. FIG. 7B is a parallel model when R5 is much larger than R2 in FIG. 4according to some embodiments of the present disclosure. As shown in FIG. 7A, the metal fabric conductive silicon composite electrode may include a base structure 701, a metal fabric electrode 720 and a conductive silicon electrode 710 arranged in sequence from top to bottom. A lower surface of the conductive silicon electrode 710 is in contact with the skin, and the metal fabric electrode 720 is between the upper surface of the conductive silicon electrode and the base structure 701 made of a material with a lower conductivity. Furthermore, with a high conductivity, the metal fabric electrode may be approximately regarded as a material transversal impedance short-circuiting the conductive silicon electrode 710 (e.g., the R2 in FIG. 4 and FIG. 7B). The metal fabric electrode helps to reduce an impedance value of an electrode parallel circuit (the parallel circuit in FIG. 4 is twice R2 plus one R1, and the parallel circuit in FIG. 7B is one R2 plus one R1), which can realize the parallel connection of more paths and greater areas, thus greatly reducing the total impedance and the contact impedance, and making a measurement result (that is, the collected EMG signal) of the electrode along the muscle fiber direction consistent. Through comparing a single conductive silicon electrode with a metal fabric conductive silicon composite electrode formed by combining the metal fabric electrode 720 and the conductive silicon electrode 710, the total impedance of the electrode is greatly reduced. In addition, due to the implementation of the parallel connection of more paths and greater area, the contact impedance between the electrode and the skin may further be effectively reduced, so that in a case of the same fluctuation, the fluctuation of a contact impedance relative to a input impedance may be reduced, thereby reducing a noise in the EMG signal. When the metal fabric conductive silicon composite electrode is used to collect the EMG signal, the conductive silicon electrode is in a direct contact with the human skin. The conductive silicon has advantages of skin-friendly and strong tolerance, which may avoid a discomfort brought to the human skin when the EMG signal collection electrode contacts the skin. In some embodiments, the thickness of the metal fabric electrode in the metal fabric conductive silicon composite electrode may be 10 $\mu$m~5 mm. According to preference for example the thickness of the metal fabric electrode in the metal fabric conductive silicon composite electrode may be 100 $\mu$m~3 mm or 500 $\mu$m~2 mm. In some embodiments, the thickness of the metal fabric electrode in the metal fabric conductive silicon composite electrode may be 1 $\mu$m~4 mm. According to preference for example, the thickness of the metal fabric electrode in the metal fabric conductive silicon composite electrode may be 10 $\mu$m~2

mm or 0.1 mm to 1 mm. In some embodiments, the width of the metal fabric conductive silicon composite electrode may be 1 mm to 5 cm. According to preference for example, the width of the metal fabric conductive silicon composite electrode may be 0.5cm~3cm. According to preference for example, the width of the metal fabric conductive silicon composite electrode may be 1 cm, 1.5 cm or 2 cm. In some embodiments, the length of the surface of the metal fabric conductive silicon composite electrode used to contact the skin may be 1 cm~10 cm. It should be noted that the length of the composite electrode may be determined according to a part measured. For example, for the upper body muscles, a composite electrode with a length of 4 cm may be used to collect the EMG signal. For another example, for a part with a great range of muscle, such as the latissimus dorsi, a composite electrode with a length of 10 cm may be used for the collection.

In some embodiments, the size (e.g., the length and width) of the metal fabric electrode is greater than the size of the conductive silicon electrode, e.g., the width of the metal fabric electrode is 1 mm~5 cm, and the width of the conductive silicon electrode is 0.8 cm~4 cm. According to preference for example, the width of the metal fabric electrode is 0.5 cm~3 cm or 1 cm~2 cm, and the width of the conductive silicon electrode is 0.4 cm~2.8 cm or 0.8 cm~1.6 cm. In some embodiments, the length of the metal fabric electrode in the metal fabric conductive silicon composite electrode may be 1 cm~10 cm. The length of the conductive silicon electrode in the metal fabric conductive silicon composite electrode may be 0.8 cm~8 cm. It should be noted that the lengths of the conductive silicon electrode and the metal fabric electrode may be determined according to the part measured. For example, for the upper body muscles, a composite electrode with a length of 4 cm may be used to collect the EMG signal, and the length of the metal fabric electrode may be 4 cm, the length of the conductive silicon electrode may be 3.2 cm. For another example, for parts with a great range of muscles, such as the latissimus dorsi, a composite electrode with a length of 10 cm may be used for the collection, the length of the metal fabric electrode may be 10 cm, and the length of the conductive silicon electrode may be 8 cm.

The movement of the limbs may cause a certain displacement or a wrinkle of the electrode relative to the skin, or even make the electrode fall off the skin, resulting in a great fluctuation in the contact impedance and even a failure to collect the EMG signal. Moreover, the displacement of the electrode may further cause a change in the position where the EMG signal needs to be collected, making the EMG signal collected inaccurate. In some embodiments, a possibility of the electrode moving relative to the skin may be reduced by increasing a tightness between the electrode and the skin. Furthermore, a close contact between the electrode and the skin may further shorten the distance between the electrode and the skin. According to the formula (1), the contact impedance between the skin and the electrode may be reduced, which is conducive to reducing the contact impedance and reducing a fluctuation of the contact impedance.

In some embodiments, to make the electrode fit to the skin tightly and reliably, the electrode module may include several protruding structures located between the electrode and a base structure, and the protruding structures may make the surface of the electrode protrude outward. In some embodiments, the protruding structures may be arranged in an array on the surface of the electrode and/or the surface of the base structure, or may further be randomly distributed. Specifically, when a certain external pressure acts on the electrode module to make the electrode contact the human skin, the protruding structures may make the electrode fit to the human skin better. On the other hand, a pressure requirement on the body of the carrier (e.g., a clothing) where the electrode module is located may be reduced by setting the protruding structures. The electrode module with the protruding structures ensures that only the electrode part has a large pressure, and the carrier does not exert much pressure on other parts of the body. While ensuring the fit of the electrode to the skin well, the user may have a better sense of comfort. In some embodiments, the electrode may be a protruding structure. For example, a central area of the electrode may be higher than an edge area of the electrode. In some embodiments, the electrode may be a planar structure, and the electrode may protrude outward relative to the base structure of the electrode module to form a protruding structure of the electrode module. In some embodiments, a height of the protruding structure may be 1 mm~10 mm. According to preference for example, the height of the protruding structure may be 2mm~8mm or 2mm~4mm. It should be noted that the center of the protruding structure may be the highest, and a periphery part of the protruding structure may be gradually lower, and the height of the protruding structure is the height of the highest position in the middle of the protruding structure. In some embodiments, the protruding structure may further be a structure where an end surface of the protruding structure in contact with the human body is approximately a planar, and the height of the protruding structure may be 0.5 mm~ 5 mm. According to preference for example, the height of the protruding structure may be 1mm~4mm or 1mm~2mm. In some embodiments, the protruding structure may further be located on the other side of the base structure, that is, the protruding structure and the electrode are distributed on different surfaces of the base structure. In some embodiments, the protruding structure may be made of an elastic material, for example, the protruding structure may be made of a soft PVC, a resin, a silicone and other materials. In some embodiments, the protruding structure may be a hollow-carved structure including a filler, and the filler may be located in the hollow part of the protruding structure. The filler may be an elastic material with a certain conductivity. For example, the filler may be an absorbent sponge, a silica gel, etc. On the one hand, the filler may remove excess moisture on the skin surface and prevent a short circuit between the electrodes.

Considering that the pressure on the protruding structures at the two electrodes is different, a deformation of the protruding structures at the two electrodes is different, which makes the contact impedance between the two electrodes and the skin different, thus making an inconsistency between the two electrodes. As a result, the collected EMG signal may be affected by the noise. In some embodiments, the noise in the EMG signal may be reduced by reducing the contact impedance. In some embodiments, the contact impedance fluctuation range may further be controlled to reduce the noise in the EMG signal. In some embodiments, the contact impedance fluctuation may be within 1M ohm. According to preference for example, the contact impedance fluctuation may be within 100K ohm or 10K ohm. The controlling the contact impedance fluctuation range may be that making sure that the contact impedance range between the two electrodes and the skin is within a specific range (e.g., within the above-mentioned 1M ohm, within 100K ohm, or within 10K ohm), or fluctuation values of the contact impedances between the two electrodes and the skin are approximately the same. In some embodiments, the noise may further be reduced through the circuit connected to the electrodes. For example, the circuit connected to the electrodes may include a differential architecture, a notch filter, a threshold filter, a right leg driver, etc. In some embodiments, the problem of inconsistent contact impedance between the two electrodes and the skin may further be solved by adjusting the structure and the height of the protruding structure. For illustrative purposes only, for example, the protruding structure is a solid structure, and the end face of the protruding structure in contact with the skin is a plane. For another example, the height of the protruding structure is 1 mm.

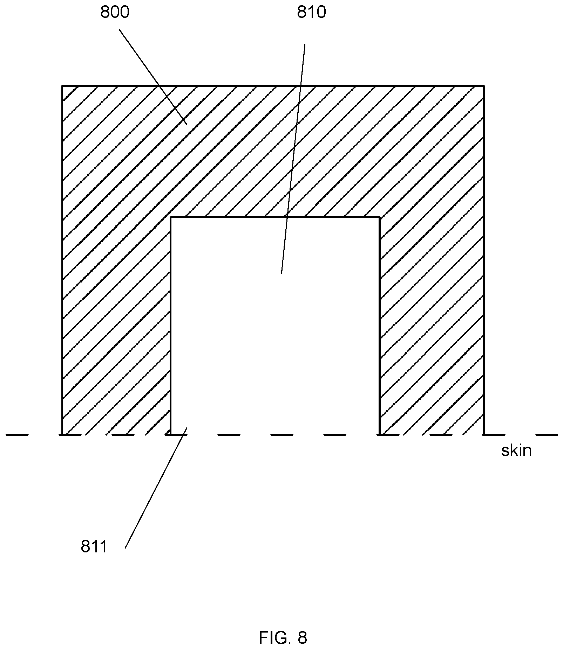

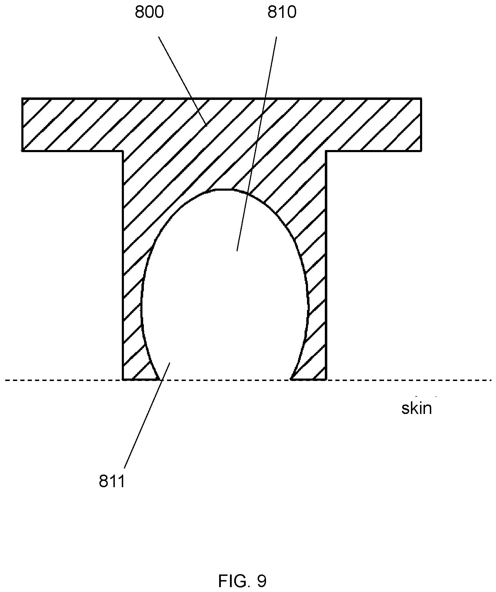

In some embodiments, the electrode module may include a plurality of sucker structures, through which the electrodes may be more closely fitted to the skin, thereby preventing the electrodes from moving when collecting the EMG signal, and ensuring the accuracy of the collected EMG signal. FIG. 8 is a cross-sectional diagram illustrating a sucker structure according to some embodiments of the present disclosure. As shown in FIG. 8, a sucker structure 800 is a regular or irregular geometric structure, such as a three-dimensional (3D) structure like a cylinder, a cone, a cuboid, etc. The sucker structure 800 may include a hollow part 810, and the hollow part has an opening 811 at an end of one end of the sucker structure. In some embodiments, at least part of the sucker structure 800 may be made of elastic materials such as a thermoplastic elastomer (TPE), a polypropylene (PPE), a polyurethane-acrylate based polymer (s-PUA), etc., so that the sucker structure 800 may be able to deform. When an electrode module is fitted to a skin, the opening 811 of the hollow part 810 in the sucker structure 800 is in contact with the human skin. Under an action of an external force, the sucker structure 800 is deformed, so that a part of the air inside the hollow part 810 is squeezed outside, and an external air pressure is greater than an internal air pressure of the hollow part 810, forming a negative pressure space with the skin surface, so that the electrode module (or the electrode) is absorbed on the skin, so as to implement an effect of the electrode fitting closely to the skin. In some embodiments, the shape of the hollow part 810 includes, but is not limited to, a sphere, an arch, a cylinder, a cube, an incomplete sphere, and other regular or irregular shapes. For example, the shape of the hollow part of the sucker structure shown in FIG. 8 is a cube. As another example, the shape of the hollow part of the sucker structure shown in FIG. 9 is an incomplete sphere.

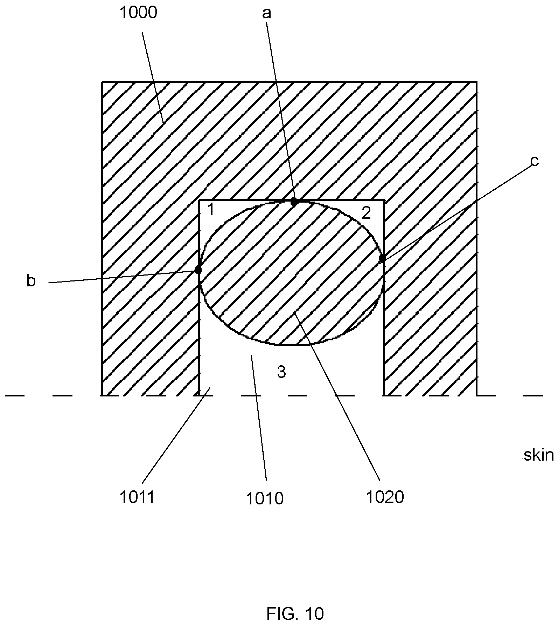

FIG. 10 is a cross-sectional diagram illustrating the sucker structure according to the present invention. The sucker structure 1000 includes a middle structure 1020 located at a hollow part 1010 of the sucker structure 1000, and a part of the middle structure 1020 is connected to a side wall of the sucker structure 1000 where the hollow part 1010 is located. The middle structure 1020 is made of soft and deformable material with a certain elasticity. For example, the middle structure 1020 may be made of a soft PVC, a silica gel, a sponge and other materials. In some embodiments, the shape of the middle structure 1020 may include, but is not limited to, a sphere, a cylinder, a cubic, and other regular or irregular shapes. In some embodiments, the middle structure 1020 may be a part of the sucker structure 1000, or may be set apart from the sucker structure 1000. The middle structure 1020 divides the hollow part 1010 into a plurality of interconnected spatial areas, thereby improving an applicability of an electrode module in a specific environment (e.g., an environment with a lot of liquid on a skin surface). For an illustration only, as shown in FIG. 10, the middle structure 1020 is connected to the sucker structure 1000, and the connection point is represented by a point a. When the middle structure is located in the hollow part 1010 of the sucker structure 1000, other parts of the middle structure 1020 (such as a point b and a point c shown in FIG. 10) are fitted to, but not connected to the side wall of the sucker structure 1000 where the hollow part 1010 is located. The fitting here is implemented by a pressing force between the elastic middle structure 1020 and the side wall of the sucker structure 1000. The middle structure 1000 may divide the hollow part into a space area 1, a space area 2, and a space area 3. Specifically, the space area 1 and the space area 3 are formed by the middle structure 1020 and the side wall of the sucker structure 1000, and the space area 3 is formed by the middle structure 1020, the side wall of the sucker structure 1000 and the human skin. The space area 1, the space area 2, and the space area 3 are connected to each other. Specifically, when the sucker structure 1000 is applied in the environment with a lot of liquid on the skin surface (e.g., the part of the human body that is prone to sweat (an armpit, a chest, etc.), the liquid (sweat) exists in the space area 3 when there is no external force. When an external force (e.g., the force generated by a muscle movement) acts on the sucker structure 1000 and pressing the sucker structure 1000, the sucker structure 1000 and the middle structure 1020 may be deformed, and the liquid in the space area 3 may reach the space area 1 and the space area 2 through the point b and the point c. After the pressing is completed, the sucker structure 1000 and the middle structure 1020 restore their shape, and part of the liquid in the space area 3 will remain in the space area 1 and the space area 2. An amount of the liquid in the space area 3 may be greatly reduced compared to the amount of liquid before the pressing, so that a strong negative pressure adsorption force may be generated between the sucker structure 1000 and the human skin.

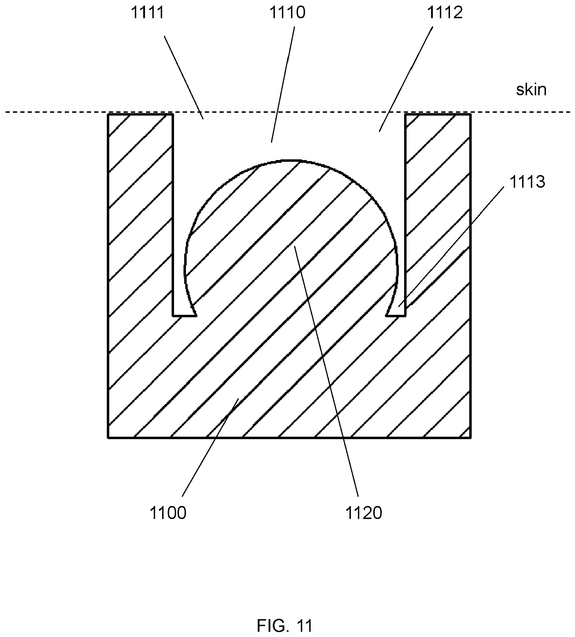

FIG. 11 is a cross-sectional diagram illustrating another sucker structure according to some embodiments of the present disclosure. As shown in FIG. 11, a body of a sucker structure 1100 has a middle part 1110, the middle part 1110 has an opening 1111 towards a skin. There is a middle structure 1120 inside the middle part 1110. Due to an existence of the middle structure 1120, a cavity may include an upper space 1112 and a lower space 1113. In some embodiments, the sucker structure 1100 may be made of an elastic material (e.g., a polyurethane-acrylate based polymer (s-PUA)), so that the sucker structure 1100 and its middle structure 1120 may deform under the action of the external force. When collecting an EMG signal, the electrode with the sucker structure 1100 may be placed on the body part where the EMG signal is to be collected, and then a pressure is applied to the sucker structure 1100 so that the sucker structure 1100 is in contact with the skin of the human body. At this time, the sucker structure 1100 works. When the sucker structure 1100 works, the upper space 1112 of the middle part 1110 of the sucker structure 1100 changes, and the lower space 1113 may also change accordingly. For example, the middle structure 1120 inside the sucker structure 1100 expands outwards, and the outer side wall contracts inward at the same time, a part of the air in the upper space 1112 and the lower space 1113 is discharged to the outside, and the external air pressure is greater than the internal air pressure of the sucker structure 1100, thus forming a negative pressure space, the electrode may be tightly and reliably adsorbed on the skin under the action of the negative pressure adsorption force of the sucker structure 1100. In some embodiments, a certain amount of phosphate buffer saline (PBS) may be loaded in the middle part 1110 of the sucker structure 1100 to improve its adsorption capacity.

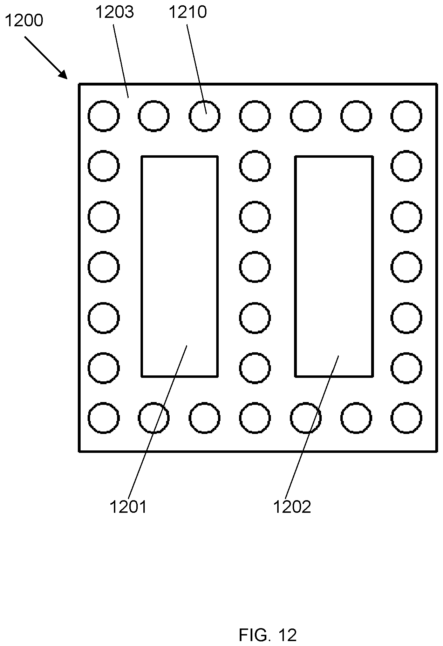

In some embodiments, FIG. 12 is a structural schematic diagram illustrating an electrode module with a sucker structure according to some embodiments of the present disclosure. As shown in FIG. 12, a sucker structure 1210 (e.g., the sucker structure 800, the sucker structure 1100) may be set on an electrode module 1200, and distributed in an array around an electrode (e.g., an electrode 1201 and an electrode 1202). The plurality of sucker structures 1210 are distributed in the array around the electrodes, and are located together with the electrodes on a side of a base structure 1203 of the electrode module 1200 facing the skin. The sucker structures 1210 and the electrodes have the same or similar height or thickness, so that when the electrode module closely and reliably adsorbed on the skin through the sucker structures, the electrodes may fully contact the skin, so as to accurately collect an EMG signal. It should be noted that the plurality of sucker structures 1210 may further be randomly distributed around the electrode. In addition, when the sucker structure 1210 is far away from the electrode, the height of the sucker structure 1210 may be greater or smaller than the height of the electrode. When the sucker structure is made of a soft and deformable material, the sucker structure 1210 may further be higher than the electrode while getting close to the electrode. After a user wears an object (e.g., a clothes) with an electrode module, it can ensure that after the pressure of the object deforms the sucker structure 1210, the contact between the electrode and the skin is not affected.

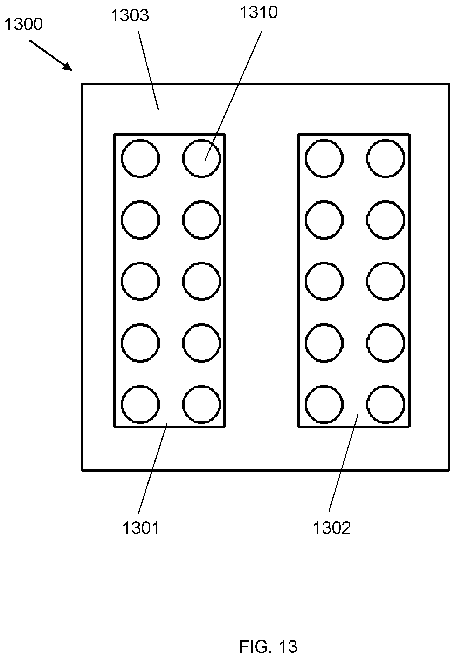

In some embodiments, the sucker structure may be arranged in an array on the electrodes, or may further be randomly distributed. FIG. 13 is a structural schematic diagram illustrating another electrode module with a sucker structure according to some embodiments of the present disclosure. As shown in FIG. 13, a plurality of sucker structures 1310 are evenly distributed on a side of the electrode (e.g., an electrode 1301 and an electrode 1302) used to contact the skin. As the electrode and the skin is connected through the sucker structure, to ensure that the electrode can collect an EMG signal, the sucker structure 1310 may be partially or entirely made of a conductive material. In some embodiments, the conductive material may include a conductive silicone, a conductive resin, a conductive plastic, etc. In this embodiment, by arranging the plurality of sucker structures 1310 in an array on a base structure 1303 of the electrode module 1300 or evenly arranging the plurality of sucker structures on the electrodes, the electrodes may be more evenly and firmly attached to the skin. In some embodiments, a plurality of sucker structures 1310 may also be randomly arranged on the base structure 1303 of the electrode module 1300 or on the electrodes.

It should be noted that the sucker structure 1210 and the sucker structure 1310 may include the sucker structures shown in FIGs. 8-11, which will not be repeated here. In other embodiments, the electrodes may further be closely and reliably fitted to the skin by ways of sticking, clamping, etc.

In some embodiments, to further prevent a relative displacement of the electrodes on the skin, the electrode module may further include several bump structures (not shown in the figure), which may increase a friction between the electrode module or the electrodes and the skin, thereby increasing an anti-skid performance of the electrode module. The bump structure may be located on the base structure or on the face of the electrode contacting the human skin. The bump structure may be in any shape, and may be in array or randomly distributed on the electrode or the base structure of the electrode module. In some embodiments, the shape of the bump structure may include regular or irregular structures such as cones, cylinders, cuboids, and hemispheres. In some embodiments, to improve the anti-skid performance of the electrode module, a height of the bump structure may be 5 $\mu$m ~ 200 $\mu$m. According to preference for example, the height of the bump structure may be 10 $\mu$m~100 $\mu$m, 20 $\mu$m ~80 $\mu$m, or 20 $\mu$m~50 $\mu$m. In some embodiments, to improve the anti-skid performance of the electrode module, a size (e.g., a length, a width or a radius, etc.) of a connection surface between the bump structure and the electrode or the base structure may be 10 $\mu$m~1000 $\mu$m. According to preference for example, the size of the connection surface between the bump structure and the electrode or the base structure may be 50 $\mu$m ~ 800 $\mu$m or 100 $\mu$m~ 600 $\mu$m. In some embodiments, to improve the anti-skid performance of the electrode module, a distribution density of the bump structures may be 1 piece/2.25mm$^2$~10 pieces/2.25mm$^2$. According to preference for example, the distribution density of the bump structures may be 3 pieces/2.25mm$^2$ to 8 pieces/2.25mm$^2$ or 5 pieces/2.25 mm$^2$ to 6 pieces/2.25 mm$^2$. As an example only, the bump structure may be randomly distributed on the conductive silicon electrode. The shape of the bump structure is conical with a height of 20~50$\mu$m and a bottom radius of 100~600$\mu$m, and the distribution density of the bump structure is 5 pieces/2.25 mm$^2$. Such a conductive silicon electrode has a good anti-skid performance. In some embodiments, the bump structure may further be arranged between the electrodes, which may block body surface fluids such as sweat between the electrodes, and prevent a short circuit of the electrodes.

As the collection of the EMG signal is usually completed when the user is exercising, during the exercise, with a consumption of heat, the sweat from the human body accumulates between the electrodes and the skin. Due to a viscosity of the sweat and the skin of human body as well as the viscosity of the sweat and the electrode, the electrode and the skin may fit too tightly, causing the skin to be unable to breathe. In this case, bacteria are easily bred. In addition, the accumulation of sweat may further cause a problem of short circuit between the electrodes. To prevent the occurrence of the above problems, the electrode module may further include a number of air holes, which may be located on the base structure of the electrode module or on the electrode to make the electrode module have a certain air permeability, thereby promoting a ventilation between the skin and the external environment, and reduce the sweat accumulation between the electrode and the human skin. In some embodiments, a shape of the air hole may be a regular or irregular shape such as a circle, an ellipse, a rectangle, a square, or a hexagon. In some embodiments, several air holes may be arranged in an array. In some embodiments, a diameter of the air hole may range from 0.2 mm to 4 mm. According to preference for example, the diameter of the air hole may range from 0.5 mm to 3 mm or 1 mm to 2 mm. According to preference for example, the diameter of the air hole may be 1mm, 1.5mm or 2mm. In some embodiments, a total area of the air holes may not exceed 90% of the electrode area. According to preference for example, the total area of the air holes does not exceed 50% or 20% of the electrode area. In some embodiments, the air holes may be made through a mechanical drilling.

The electrode module provided by the embodiment of the present disclosure may not only be integrated on the clothing or other wearable apparatuses to effectively collect the EMG signals of various parts of the user's body during exercise. It may further be configured in a medical rehabilitation engineering to collect the EMG signals of patients, so as to make corresponding diagnosis or treatment for the patients. Or in bionics, the electrode module may be configured to collect the EMG signal for research on artificial limbs.

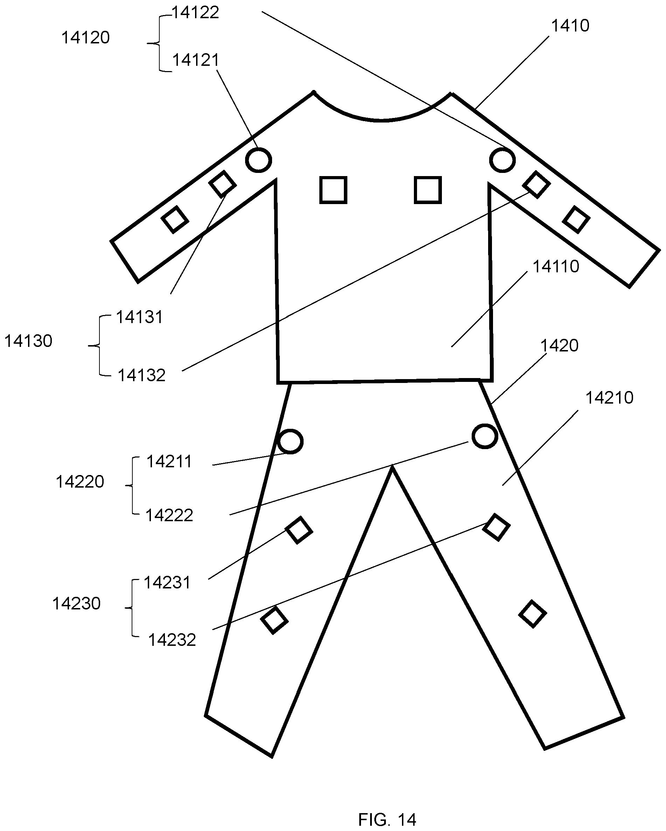

FIG. 14 is a structural schematic diagram illustrating an exemplary wearable apparatus according to some embodiments of the present disclosure. As shown in FIG. 14, a wearable apparatus 1400 may include an upper garment 1410 and pants 1420. The upper garment 1410 may include an upper garment base 14110, at least one upper garment data processing module 14120, at least one upper garment sensor module 14130, etc. The at least one upper garment data processing module 14120 and the at least one upper garment sensor module 14130 are located in areas fitting different parts of the human body on the upper garment base 14110. The upper garment base 14110 may refer to a clothing worn on the upper body of a human body. In some embodiments, the upper garment base 14110 may include a short-sleeved T-shirt, a long-sleeved T-shirt, a shirt, a coat, etc. Referring to FIG. 14, the at least one upper garment data processing module 14120 may include a first upper garment data processing module 14121 and a second upper garment data processing module 14122. Specifically, the first upper garment data processing module 14121 may be arranged at a left shoulder of the upper garment base 14110, and the second upper garment data processing module 14122 may be arranged at a right shoulder of the upper garment base 14110. It should be noted that the first upper garment data processing module 14121 and the second upper garment data processing module 14122 may be symmetrically distributed along a midline of the upper garment base 14110 or asymmetrically distributed along the midline of the upper garment base 14110. The at least one upper garment sensor module 14130 may include a first upper garment sensor module 14131 and a second upper garment sensor module 14132. The first upper garment sensor module 14131 may include one or more sensors of the same or different types, for example, an EMG signal sensor (also referred to as an EMG signal module), an electrocardiogram (ECG) sensor, a respiration sensor, a temperature sensor, a humidity sensor, etc. The first upper garment sensor module 14131 may be set on a muscle position left of the upper garment base 14110 (e.g., on a left arm biceps, a left arm wrist extensor, a left pectoralis major, etc.). The second upper garment sensor module 14132 may refer to one or more sensors of the same or different types, for example, an EMG signal sensor, an ECG sensor, a respiration sensor, a temperature sensor, a humidity sensor, etc. The second upper garment sensor module 14132 may be set on a muscle position right of the upper garment base 14110 (e.g., a right arm biceps, a right arm wrist extensor, a right pectoralis major, etc.). In some embodiments, the first upper garment data processing module 14121 and the first upper garment sensor module 14131 may be connected through an electrode wiring, and the second upper garment data processing module 14122 and the second upper garment sensor module 14132 may be connected through the electrode wiring. In some embodiments, the first upper garment data processing module 14121 and the first upper garment sensor module 14131 may be connected by a wireless communication, and the second upper garment data processing module 14122 and the second upper garment sensor module 14132 may be connected by the wireless communication. For example, the human body EMG signal collected by the EMG signal sensor of the first upper garment sensor module 14131 may be transmitted to the first upper garment data processing module 14121 in a wired or a wireless mode. It should be noted that for a specific content of the EMG signal sensor (the electrode module), please refer to the description elsewhere of the present disclosure (e.g.,

FIG. 1-FIG. 3, FIG. 7-FIG. 13), which will not be repeated here.

In some embodiments, the first upper garment data processing module 14121 may be set at the left shoulder of the upper garment base 14110, and the first upper garment data processing module 14121 is configured to receive upper body moving data of a left sensor of the human body (e.g., an EMG signal sensor, an electrocardiographic sensor, a breathing sensor, etc.). The second upper garment data processing module 14122 may be set at the right shoulder of the upper garment base 14110, and the second upper garment data processing module 14122 is configured to receive upper body moving data of a right sensor of the human body (e.g., an EMG signal sensor, an electrocardiographic sensor, a breathing sensor, etc.). The aforementioned first upper garment data processing module 14121 at the left shoulder and the second upper garment data processing module 14122 at the right shoulder may be symmetrically distributed along the midline of the upper garment base 14110.

In some embodiments, the first upper garment data processing module 14121 at the left shoulder and the second upper garment data processing module 14122 at the right shoulder may be in a subordination. For example, the first upper garment data processing module 14121 is a master data processing module, and the second upper garment data processing module 14122 is a subordinate data processing module. At this time, the first upper garment data processing module 14121 and the second upper garment data processing module 14122 may be communicatively connected by the wired and the wireless modes. Specifically, the first upper garment data processing module 14121 receives the moving data of the left side of the human body, the second upper garment data processing module 14122 receives the moving data of the right side of the human body, and the second upper garment data processing module 14122 may transmit the received moving data of the right side of the human body to the first upper garment data processing module 14121 for processing. In some embodiments, the wiring mode between the first upper garment data processing module 14121 and the second upper garment data processing module 14122 may be a wire wired on the front of the wearable apparatus 1400 or on the back of the wearable apparatus 1400. In some embodiments, the first upper garment data processing module 14121 may further transmit the moving data of the left side of the human body received by itself and the right moving data of the human body received from the second upper garment data processing module 14122 to a peripheral terminal. The peripheral terminal performs a data processing on the received moving data.

In some embodiments, the first upper garment data processing module 14121 and the second upper garment data processing module 14122 may further be in a parallel. When the first upper garment data processing module 14121 and the second upper garment data processing module 14122 are in a parallel, the first upper garment data processing module 14121 and the second upper garment data processing module 14122 may be communicatively connected to the peripheral terminals respectively. Specifically, the first upper garment data processing module 14121 receives the moving data from the left side of the human body, and transmits the received moving data to the peripheral terminal, and the peripheral terminal processes the received moving data. The second upper garment data processing module 14122 receives the moving data from the right side of the human body, and transmits the received moving data to the peripheral terminal, and the peripheral terminal processes the received moving data.

In some embodiments, the moving data of the human body received by the upper garment data processing module 14120 may be processed by the upper garment data processing module 14120. For example, the moving data of the left side of the human body received by the first upper garment data processing module 14121 may be processed in the first upper garment data processing module 14121, and the first upper garment data processing module 14121 may transmit the processed moving data to the peripheral terminal. In some embodiments, the human body moving data received by the upper garment data processing module 14120 may further be processed in the peripheral terminal. For example, the second upper garment data processing module 14122 may directly transmit the received moving data of the right side of the human body to the peripheral terminal, and the peripheral terminal may process the received moving data of the right side of the human body. In some embodiments, the human body moving data received by the upper garment data processing module 14120 may further be processed partly in the upper garment data processing module 14120, and partly in the peripheral terminal. For example, the moving data on the left side of the human body received by the first upper garment data processing module 14121 may be partly processed in the first upper garment data processing module 14121, and the first upper garment data processing module 14121 transmits the partly processed moving data to the peripheral terminal. The peripheral terminal then performs processing on the other part of data which is not processed yet.

In some embodiments, there may be a mutual communication between the garment data processing module 14120 and the peripheral terminal. For example, the first upper garment data processing module 14121 (or the second upper garment data processing module 14122) may transmit the moving data to the peripheral terminal, and may further receive an instruction from the peripheral terminal. For example, the instruction may be an instruction to control an operation of the electrode module, or an instruction to perform a moving quality feedback according to the user's moving data.

Continuing to refer to FIG. 14, the pants 1420 may include a pants base 14210, at least one pants data processing module 14220, at least one pants sensor module 14230, etc. The at least one pants data processing module 14220 may include a first pants data processing module 14221 and a second pants data processing module 14222. Specifically, the first pants data processing module 14221 may be set at a crotch on the left of the pants base 14210, and the second pants data processing module 14222 may be set at a crotch on the right of the pants base 14210. It should be noted that the first pants data processing module 14221 and the second pants data processing module 14222 may be distributed symmetrically along the midline of the pants base 14210, or may be distributed asymmetrically along the midline of the pants base 14210. The at least one pants sensor module 14230 may include a first pants sensor module 14231 and a second pants sensor module 14232. The first pants sensor module 14231 may include one or more different types of sensors, for example, an EMG signal sensor, a temperature sensor, a humidity sensor, etc. The first pants sensor module 14231 may be arranged on a left muscle position of the left leg of the pants base 14210 (e.g., a left gluteus maximus, a left vastus lateralis, a left vastus medialis, a left biceps femoris, etc.). In some embodiments, the first pants sensor module 14231 may be set at the muscle position of the front of the left leg and the muscle position of the back of the left leg of the pants base 14210. The second pants sensor module 14232 may include one or more sensors of different types, for example, the EMG signal sensor, the temperature sensor, the humidity sensor, etc. The second pants sensor module 14232 may be set at the muscle position of the right of the right leg of the pants base 14210 (e.g., a right gluteus maximus, a right vastus lateralis, a right vastus medialis, a right biceps femoris, etc.). In some embodiments, the second pants sensor module 14232 may be set at the muscle position of the front of the right leg and the muscle position of the back of the right leg of the pants base 14210. In some embodiments, the first pants data processing module 14221 and the first pants sensor module 14231 may be connected through the electrode wiring, and the second pants data processing module 14222 and the second pants sensor module 14232 may be connected through the electrode wiring. For contents on the at least one pants data processing module 14220 (e.g., the first pants data processing module 14221 and the second pants data processing module 14222), and the at least one pants sensor module 14230 (e.g., the first pants sensor module 14231 and the second pants sensor module 14232 ), please refer to the relevant descriptions of the at least one upper garment data processing module 14120 and the at least one upper garment sensor module 14130, which will not be repeated here.

In some embodiments, the sensor module (e.g., the first upper garment sensor module 14131, the second upper garment sensor module 14132, the first pants sensor module 14231, the second pants sensor module 14232) may include, but are not limited to, an EMG signal sensor, an inertial sensor, an ECG sensor, a respiratory sensor, a temperature sensor, a humidity sensor, an acid-base sensor, an acoustic transducer, etc.

In some embodiments, the EMG signal sensor (also referred to as the electrode module) may be set at the muscle position of the human body. The EMG signal sensor and the data processing modules (e.g., the first upper garment data processing module 14121, the second upper garment data processing module 14122, the first pants data processing module 14221, the second pants data processing module 14222) may communicate with each other. The muscle position corresponding to the upper garment human body may include but not limited to a biceps, a triceps, a wrist extensor, a wrist flexor, a deltoid (such as a front deltoid, a middle deltoid, and a back deltoid), a trapezius, latissimus dorsi, a pectoralis major, an external oblique, a rectus abdominis, etc. The muscle position corresponding to the pants may include but not limited to a gluteus maximus, a vastus lateralis, a vastus medialis, a rectus femoris, a biceps femoris, tibialis anterior, a gastrocnemius, etc. In some embodiments, the EMG signal sensor may be configured to collect muscle information of the user during the moving, such as a moving range, a moving speed, a moving strength, etc. For example, when the user performs a barbell bench press, the EMG signal sensor set on the biceps (or the triceps, the deltoid, the pectoralis major, etc.) may collect muscle information (such as the moving range, the moving speed, the moving strength, etc.), and transmit the collected muscle information to the data processing module. The data processing module performs the data processing on the received muscle information (or the data processing module transmits the received muscle information to the peripheral terminal, and the peripheral terminal performs the data processing on the received muscle information), and determines whether the muscle data of the user in the barbell bench press process is within the determination standard, in response to the determination that the data exceeds the determination standard, the wearable apparatus may warn the user through a feedback module. The determination standard here may be obtained in advance through training the muscle data of professionals (e.g., athletes, coaches, etc.) and a large number of ordinary people in the processes of barbell bench press, which is recorded in the wearable apparatus. At the same time, the determination standard may be adjusted according to user information to achieve a better adaptability. According to the warning of the feedback module, the user may know in real time whether their barbell bench press muscle state (e.g., the moving range, the moving speed, the moving strength, etc.) is standardized, and adjust the moving posture in time.

In some embodiments, the inertial sensor may be integrated in the sensor module, in this case, the inertial sensor may be separated from the data processing module, thus causing practical problems. For example, a washing issue, a complexity issue (e.g., a separate fabrication, a disassembly, etc.), a power supply issue, a charging issue, etc. In some embodiments, the inertial sensor may further be set in a left data processing module and a right data processing module of the garment base, and the inertial sensor may be configured to monitor the user's moving parameters (such as a stride number, a stride length, a stride frequency, etc.). Specifically, the inertial sensor may be set in the left data processing module and the right data processing module of the garment base. On the one hand, the inertial sensor may monitor the user's moving parameters, such as the stride number, the stride length, and the stride frequency, etc. On the other hand, a problem of the inertial sensor separating from the data processing module (such as washing problems, power supply problems, etc.) may be effectively solved. Moreover, the inertial sensors respectively set in the first data processing module and the second data processing module may further monitor a moving coordination and a balance of the left and right sides of the user's body.

In some embodiments, more inertial sensors may be designed inside and/or outside the data processing module to realize more complex functions such as an action identification. For example, an acceleration sensor may be configured to collect an acceleration when the human body moves. Specifically, an acceleration measurement may be mainly used for a step count and a calorie calculation during the user's moving process, and may further be used to monitor the acceleration of the user's body in three axes of X, Y and Z during the user's moving process.

In some embodiments, the ECG sensor may be configured to collect the ECG data during the user's moving. The ECG sensor and the data processing module (e.g., the first upper garment data processing module 14121 and the second upper garment data processing module 14122) may be communicatively connected. When a user is exercising, its heart rate may change. For example, when the user is doing an aerobic exercise or a strength training, a duration of the user's exercise and/or an intensity of the exercise may affect the user's heart rate. The ECG sensor may collect an ECG signal during the user's exercise, and transmit the collected ECG signal to the data processing module. The data processing module performs the data processing on the received ECG signal (or the data processing module transmits the received ECG signal to the peripheral terminal, and the peripheral terminal performs the data processing on the received ECG signal), and determines whether the user's heart rate is within the determination standard, in response to that the heart rate exceeds the determination standard, the wearable apparatus may warn the user through the feedback module. The determination standard here may be obtained in advance through training the ECG data of the professionals (e.g., athletes, coaches, etc.) and a large number of ordinary people in the processes of exercises, which is recorded into the wearable apparatus. At the same time, the determination standard may be adjusted according to user information to achieve a better adaptability. According to the warning of the feedback module, the user may know in real time whether their ECG state is normal, and adjust the exercise duration and/or the exercise intensity in time.

In some embodiments, the respiration sensor may be configured to collect a respiration signal during the user's exercise. The respiration sensor may be communicatively connected to the data processing module (e.g., the first upper garment data processing module 14121 and the second upper garment data processing module 1412). During the exercise, the user's respiration rate and respiration frequency may change. For example, when the user is running, the respiration rate may gradually increase with a lengthen of the user's running duration and/or an increase of a running speed, and the respiration frequency may increase with the lengthen of the user's running duration and/or the increase of the running speed. The respiration sensor collects the respiration signal (such as a respiration speed, a respiration frequency, etc.) of the user during the exercise, and transmits the collected respiration signal to the data processing module. The data processing module performs a data analysis on the received respiration signal (or the data processing module transmits the received respiration signal to the peripheral terminal, and the peripheral terminal performs the data processing on the received respiration signal), and determines whether the user's respiration data during the running process is within the determination standard, in response to a determination that the respiration data exceeds the determination standard, the wearable apparatus may warn the user through the feedback module. The determination standard here may be trained in advance through training the respiration data of the professionals (e.g., athletes, coaches, etc.) and a large number of ordinary people in the process of running, which is recorded into the wearable apparatus. At the same time, the determination standard may be adjusted according to the user's information to achieve a better adaptability. According to the warning of the feedback module, the user may know in real time whether their running state (e.g., a running speed, a running duration, etc.) is standardized, and adjust the running duration and/or the running speed in time.

In some embodiments, the temperature sensor may be configured to collect a temperature signal during the user's exercise. The temperature sensor may communicatively connect the data processing module (e.g., the first upper garment data processing module 14121, the second upper garment data processing module 14122, the first pants data processing module 14221, and the second pants data processing module 14222). During exercise, the user's body temperature may gradually increase. For example, when the user is doing the aerobic exercise or the strength training, the duration of the user's exercise and/or the intensity of the exercise may affect the user's body temperature. The temperature sensor may collect the temperature signal during the user's exercise, and transmit the collected temperature signal to the data processing module. The data processing module performs the data processing on the received temperature signal (or the data processing module transmits the received temperature signal to the peripheral terminal, and the peripheral terminal performs the data processing on the received temperature signal), and determines whether the user's body temperature is within the determination standard; in response to a determination that the body temperature exceeds the determination standard, the wearable apparatus may warn the user through the feedback module. The determination standard here may be trained in advance through training the temperature data of the professionals (e.g., athletes, coaches, etc.) and a large number of ordinary people in the process of exercising, which is recorded into the wearable apparatus. At the same time, the determination standard may be adjusted according to the user's information to achieve a better adaptability. According to the warning of the feedback module, the user may know in real time whether their temperature state is normal, and adjust the exercise duration and/or the exercise speed in time.

In some embodiments, the humidity sensor may be configured to collect a humidity signal during the user's exercise. The humidity sensor may communicatively connect the data processing module (e.g., the first upper garment data processing module 14121, the second upper garment data processing module 14122, the first pants data processing module 14221, and the second pants data processing module 14222). During the user's exercise, the humidity of its body may gradually increase. For example, when the user is doing the aerobic exercise or the strength training, the duration of the user's exercise and/or the intensity of the exercise may affect the user's body humidity. The humidity sensor may collect the humidity signal during the user's exercise, and transmit the collected humidity signal to the data processing module. The data processing module performs the data processing on the received humidity signal (or the data processing module transmits the received humidity signal to the peripheral terminal, and the peripheral terminal performs the data processing on the received humidity signal), and determines whether the user's body humidity is within the determination standard, in response to a determination that the body humidity exceeds the determination standard, the wearable apparatus may warn the user through the feedback module. The determination standard here may be trained in advance through training the humidity data of the professionals (e.g., athletes, coaches, etc.) and a large number of ordinary people in the process of exercising, which is recorded into the wearable apparatus. At the same time, the determination standard may be adjusted according to the user's information to achieve a better adaptability. According to the warning of the feedback module, the user may know in real time whether their humidity state is normal, and adjust the exercise duration and/or the exercise intensity in time.

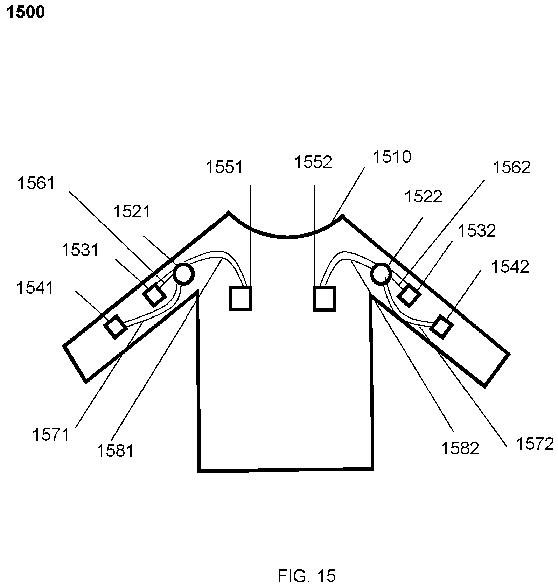

FIG. 15 is a structural diagram illustrating an exemplary wiring connection mode of an upper garment wearable apparatus according to some embodiments of the present disclosure. As shown in FIG. 15, an upper garment wearable apparatus 1500 may include a garment base 1510, a first data processing module 1521, a second data processing module 1522, a first EMG signal sensor 1531, a second EMG signal sensor 1532, a temperature sensor 1541, a humidity sensor 1542, a first ECG sensor 1551, a second ECG sensor 1552, a connection line 1561 (or a connection line 1562, a connection line 1571, a connection line 1572, a connection line 1581, a connection line 1582, etc.), etc. Specifically, the first EMG signal sensor 1531 communicatively connects to the first data processing module 1521 through the connection line 1561. The second EMG signal sensor 1532 communicatively connects to the second data processing module 1522 through the connection line 1562. The temperature sensor 1541 communicatively connects to the first data processing module 1521 through the connection line 1571. The humidity sensor 1542 communicatively connects to the second data processing module 1522 through the connection line 1572. The first ECG sensor 1551 communicatively connects to the first data processing module 1521 through the connection line 1581. The second ECG sensor 1552 communicatively connects to the second data processing module 1522 through the connection line 1582.

In some embodiments, the first data processing module 1521 is set on the left shoulder of the upper wearable apparatus 1500, and the second data processing module 1522 is set on the right shoulder. The first data processing module 1521 and the second data processing module 1522 are respectively configured to receive moving data of the left and right sides of the user's body. On the one hand, such design may effectively share a wiring route and reasonably control a volume of a single data processing module (e.g., the first data processing module 1521 and a second data processing module 1522). On the other hand, the design of setting the data processing modules on the left and right sides of the upper garment wearable apparatus 1500 is symmetrical, which may effectively solve a problem of user safety brought by an asymmetrical design (e.g., a balance control of sports like squatting). On the other hand, the design of setting the data processing modules on the left and right sides of the upper garment wearable apparatus 1500 may effectively avoid an existence of electronic device including a circuit (e.g., a battery, a Bluetooth, an amplifier, etc.), which improves the user's wearing comfort. In some embodiments, the design of setting the data processing modules on the left and right sides of the upper garment wearable apparatus 1500 may reduce a design difficulty and a weight of each data processing module, and may further effectively collect a physiological signal near the chest (e.g., an ECG signal, an EMG signal of a pectoralis major, etc.).

In some embodiments, when there are many sensor modules on the upper garment wearable apparatus 1500, the above wiring mode may effectively separate a wiring layout on the one hand, thereby reducing a wiring difficulty, and increasing a design space. On the other hand, a large-angle wiring may be avoided. In some embodiments, a wiring length in the wearable apparatus may affect its SNR, specifically, the greater the wiring length is, the greater a noise interference introduced is. The wiring mode in FIG. 15 may effectively shorten the wiring length, thereby reducing a noise signal collected by the sensor module and reducing the noise interference. In some embodiments, the sensor modules are all communicatively connected to the data processing module, and the wiring in the wearable apparatus is relatively dense, and a mutual interference may occur between various channels. The wiring mode in FIG. 15 may effectively increase distances between the wirings and reduce the mutual interference between channels.

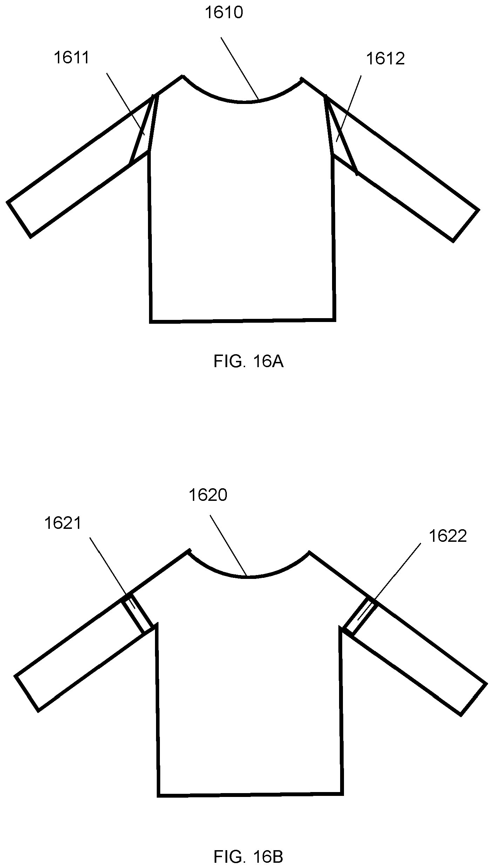

In some embodiments, during the user's exercise, most of the exercises (e.g., a barbell bench press, a dumbbell press, a running, a chest expansion, etc.) require a participation of human limbs, and the movement of the arms can drive the wearable apparatus to displace, so that the sensor module in the wearable apparatus may be displaced, and a quality of signal collection (e.g., the EMG signal at the pectoralis major muscle, the ECG signal, etc.) may be reduced. FIG. 16A is a structural diagram illustrating an exemplary elastic design at an underarm position of an upper garment wearable apparatus (also referred to as an upper garment of a wearable apparatus) according to some embodiments of the present disclosure. As shown in FIG. 16A, a first elastic design 1611 is set on a left underarm of a garment base 1610, and a second elastic design 1612 is set on a right underarm of the garment base 1610. FIG. 16B is a structural diagram illustrating an exemplary elastic design at an upper arm position of an upper garment wearable apparatus according to some embodiments of the present disclosure. As shown in FIG. 16B, a third elastic design 1621 is set on a left arm of a garment base 1620, and a fourth elastic design 1622 is set on a right arm of the garment base 1620. The third elastic design 1621 and the fourth elastic design 1622 are elastic designs of an arm ring. It should be noted that the setting positions of the elastic design may include but not limited to the underarm position and the upper arm position. This embodiment only describes the underarm position and the upper arm position. Other human body positions (such as an elbow position, a shoulder position etc.) will not be described in detail here.

In some embodiments, the elastic design may refer to a use of highly elastic and stretchable materials. The highly elastic stretchable material may include but not limited to an elastic fiber fabric material, a flexible polymer material, an elastic rubber ring, etc. In addition, the material used for the elastic design may be a single elastic material, or may be a combination of various elastic materials. For example, the garment base at the upper arm position of the wearable apparatus (e.g., the third elastic design 1621 in FIG. 16B or the fourth elastic design 1622 in FIG. 16B) may be made of an elastic rubber material. When the user's upper arm position is pulled, the highly elastic rubber material may be stretched, thereby effectively avoiding the displacement of the wearable apparatus. As another example, the garment base at the underarm area of the wearable apparatus (e.g., the first elastic design 1611 in FIG. 16A or the second elastic design 1612 in FIG. 16A) may be made of a composite material of elastic fiber fabric insert pieces and a nylon (or a polyester stretch material) material. Specifically, when the user stretches the arm, the underarm area of the wearable apparatus is pulled, and the composite material of elastic fiber fabric inserts and a nylon (or a polyester stretch material) material has a better stretch rate and recovery rate, which effectively avoids the displacement of the wearable apparatus.

In some embodiments, the elastic design may further refer to a structural design. Specifically, a wrinkled and undulating structure may be designed at the upper arm position of the wearable apparatus close to the shoulder of the human body (please refer to the third elastic design 1621 and the fourth elastic design 1622 in FIG. 16B). The wrinkled and undulating structure protrudes in a circular 3D mode relative to the wearable apparatus itself, and a magnitude of a protrusion displacement may be determined according to the magnitude of the pulling force during the moving. The wrinkled and undulating structure may be stretched along a pulling direction when the upper arm is pulled by the user's movement (e.g., a barbell bench press), which effectively alleviates the displacement of the wearable apparatus due to the pulling and affects the quality of the signal collection.

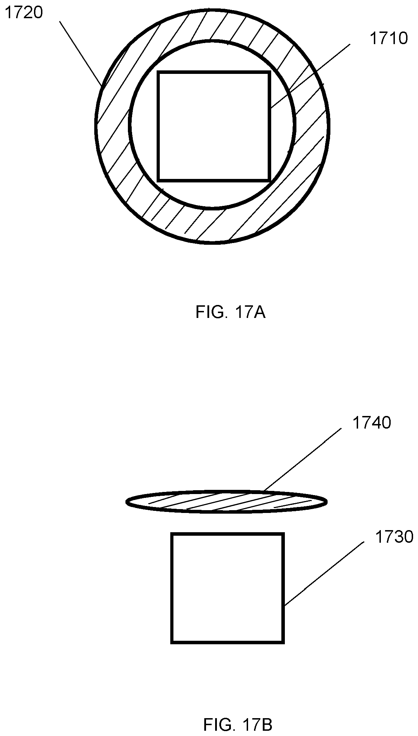

In some embodiments, the elastic design can be made for the periphery of the electrode module according to an analysis of the pulling force near the electrode. FIG. 17A is a structural diagram illustrating an exemplary annular elastic design of an electrode module of a wearable apparatus according to some embodiments of the present disclosure. As shown in FIG. 17A, an annular elastic design 1720 (e.g., using a highly elastic stretchable material, a wrinkled design, etc.) is set on the periphery of an electrode module 1710, which may reduce a pulling of the electrode module by a user in all directions during an exercise. For example, when a user wearing a sports device stretches its arms around, the electrode module 1710 on its chest and the vicinity may be pulled from different angles. In this case, setting the annular elastic design 1720 (e.g., using a highly elastic stretchable material, a wrinkled design, etc.) on the periphery of the electrode module 1710 may effectively avoid the displacement of the electrode module 1710 caused by the user's pulling, and further weaken the effect of the user's movement on the signal collection module. FIG. 17B is a structural diagram illustrating an exemplary direction-specific elastic design of an electrode module of a wearable apparatus according to some embodiments of the present disclosure. As shown in FIG. 17B, an elastic design 1740 (e.g., using the highly elastic stretchable material, the wrinkled design, etc.) in one or more specific directions of the electrode module 1730 may weaken the pulling in the one or more specific directions. For example, when a user wearing the sports device performs a chest expansion exercise, the electrode module 1730 on the chest may be pulled by the side close to the arm of the same side. In this case, setting the elastic design 1740 (e.g., using the highly elastic stretchable material, the pleated design, etc.) in the direction of the chest electrode module 1730 close to the arm on the same side may effectively avoid the displacement of the electrode module 1730 caused by the user's pulling, and further weaken the effect of the user movement on the signal collection module.

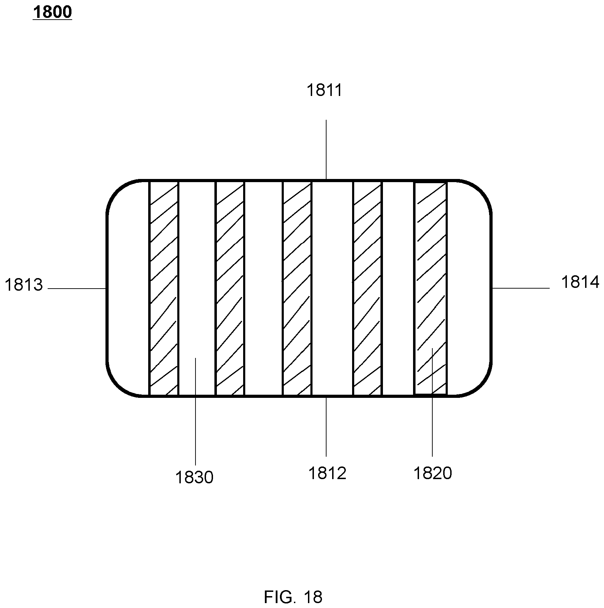

FIG. 18 is a structural diagram illustrating an exemplary hollow-carved design of a wearable apparatus according to some embodiments of the present disclosure. As shown in FIG. 18, a part of a garment base 1800 may include a first side end 1811, a second side end 1812, a third side end 1813, a fourth side end 1814, a hollow-carved design garment base 1820 (a shaded part in FIG. 18), a conventional garment base 1830 (a blank strip-shaped part in FIG. 18), etc. Here, the part of the garment base 1800 may be a structure of a partial area of the garment, and the first side end 1811, the second side end 1812, the third side end 1813 and the fourth side end 1814 may be respectively connected to the garment. The first side end 1811 is set opposite to the second side end 1812. When a pulling force is generated along a direction from the first side end 1811 to the second side end 1812, the wearable apparatus with the hollow-carved design may have a better extensibility than a conventional wearable apparatus. Continuing to refer to FIG. 18, the third side end 1813 is set opposite to the fourth side end 1814. When the pulling force is generated along a direction from the third side end 1813 to the fourth side end 1814, the hollow-carved structure becomes greater under the pulling force, making the wearable apparatus with the hollow-carved design have better extensibility than the conventional wearable apparatus. In some embodiments, when a user wears a sports device performs a chest expansion exercise, the garment at the user's chest may be displaced due to the movement. The hollow-carved structure is set around the chest of the wearable apparatus. The hollow-carved structure may effectively weaken the displacement of the electrode relative to the skin caused by the pulling of the user's movement, thereby effectively improving a quality of a signal collection at the user's chest. Referring to the hollowed-carved design mode shown in FIG. 18, on the one hand, the garment of the same material may have better extensibility, and may effectively enhance the stretchability of the garment. On the other hand, the mode may make the garment have a better breathability and an aesthetic advantage. On the other hand, the hollow-carved design may avoid a splicing of different materials, and may easily save a design cost. It should be noted that the hollow-carved structure may be set in a plurality of positions of the garment base, which is not limited in the present disclosure. Shapes of the part of the garment base 1800 and the hollow-carved garment base 1820 are not limited to the rectangular shape shown in FIG.18. The part of the garment base 1800 and the hollow-carved garment base 1820 may further be triangles, circles, parallelograms, diamonds and other regular or irregular shapes. In addition, the side ends that are used to connect the garment are not limited to the above first side end 1811, second side end 1812, third side end 1813 and fourth side end 1814, and may be adjusted according to the shape of the part of the garment base 1800 to achieve a better adaptability。

In some embodiments, the garment base of the wearable apparatus may be designed with an extra elasticity. The extra elastic design may be achieved through a 3D printing and other processes. It may be a strip of extra material attached to the garment base, or it may be a material splicing and/or the structural design of the garment base itself. For the related descriptions about the clothing materials and the structural design, please refer to FIG. 16A, which will not be repeated in the embodiment. In some embodiments, a local pressure may be designed near the electrodes in the wearable apparatus, which may effectively enhance an adhesion force near the electrodes to the human body, and at the same time, such design does not increase a pressure of the rest of the wearable apparatus on the human body, and does not affect the user's wearing comfort. For example, a pressurization design may be performed on the arm joints of the wearable apparatus. When the user is exercising, its arm may be pulled. In this case, the pressurize design on the arm joints may effectively reduce the displacement of the garment base on the user's body. In some embodiments, a local pressurization may be designed near the electrodes in the wearable apparatus, which may further effectively enhance a consistency of the two electrodes on the electrode module. Specifically, a surface of the human body is an irregular curved face, and the same electrode module arranged in the garment base and fitted to the surface of the human body may further face different fitting pressures. In this case, the local pressurization design near the electrodes in the wearable apparatus may effectively adjust a fitting pressure balance of the two electrodes of the electrode module.

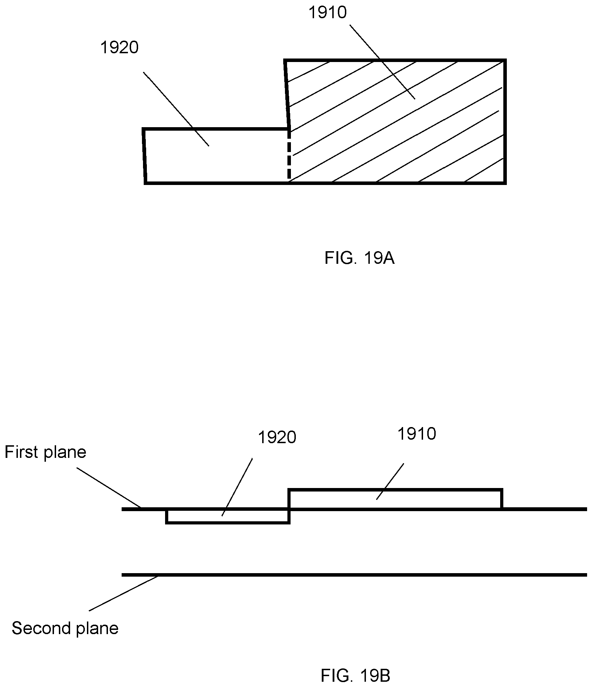

In some embodiments, external factors such as a human body surface and a human body movement may affect the electrode modules in the garment base. To effectively avoid the effect of operations such as a wire transfer, a soft to hard switch, etc. on the consistency of the electrodes, the embodiment provides a knife-shaped electrode. FIG. 19A is a structural diagram illustrating an exemplary knife-shaped electrode module according to some embodiments of the present disclosure. As shown in FIG. 19A, the knife-shaped electrode may include a first electrode part 1910 (corresponding to a shaded part in FIG. 19A) and a second electrode part 1920 (corresponding to the blank part in FIG. 19A). The first electrode part 1910 and the second electrode part 1920 are connected with each other, the first electrode part 1910 is configured to contact a human skin, and the second electrode part 1920 is configured to switch a data wire so as to transmit an EMG signal collected by the first electrode part 1910. In some embodiments, an upper surface of the second electrode part 1920 may not be in contact with the user's skin. Furthermore, the first electrode part 1910 and the second electrode part 1920 have a certain height difference. When the first electrode part 1910 is in contact with the skin, there is a certain distance between the second electrode part 1920 and the human skin. Through switching the data line at the second electrode part 1920, an interference of the second electrode part 1920 on the EMG signal collection of the first electrode part 1910 may be prevented. In some embodiments, a part of the upper surface of the second electrode part 1920 may be in contact with the user's skin. In some embodiments, a lower surface of the first electrode part 1910 and the lower surface of the second electrode part 1920 may be on the same plane. At this time, a thickness of the first electrode part 1910 is greater than the thickness of the second electrode part 1920, so that when the first electrode part 1910 contacts the user's skin, there is a certain distance between the second electrode part 1920 and the user's skin. In some embodiments, the lower surface of the first electrode part 1910 and the lower surface of the second electrode part 1920 may not be on the same plane. For example, the lower surface of the first electrode part 1910 may be located above the lower surface of the second electrode part 1920. For another example, the lower surface of the second electrode part 1920 may be located above the first electrode part, but the upper surface of the second electrode part 1920 may be below the upper surface of the first electrode part 1910, so that when the first electrode 1910 contacts the user's skin, there is a certain distance between the second electrode part 1920 and the user's skin. FIG. 19B is a cross-sectional view of an exemplary knife-shaped electrode module according to some embodiments of the present disclosure. As shown in FIG. 19B, for the convenience of illustration and description, and only for an exemplary explanation, the knife-shaped electrode structure is applied to a garment. A lower surface of the first electrode part 1910 is connected to a first plane of an inner layer of the garment, and an upper surface of the second electrode part 1920 is connected to the first plane, so that the second electrode part 1920 is located between the first plane and the second plane. Here, the first plane and the second plane may be the inner and outer sides of the garment or the inner and outer sides of other structures connected to the garment. Both the first plane and the second plane are insulating materials. For example, the insulating material may refer to a polyester, a cotton cloth, a rubber, an aramid fiber, etc., which is not limited in this embodiment.

In some embodiments, increasing a size of the electrode may effectively avoid the electrode from falling off due to a user's movement, and at the same time, ensure that a sensor can collect a signal as much as possible. For the material and size of the knife-shaped electrode, please refer to the descriptions elsewhere in the present disclosure (e.g., FIG. 3).

In some embodiments, the second electrode part 1920 in FIG. 19A may be an area where a conduction signal is transferred away from the first electrode part 1910. Most of the electrodes in the wearable apparatus are flat, and the flat electrodes are directly used for the signal conduction after cutting, which occupies a relatively great space. Moreover, an impedance of a signal conduction route is great, which is not conducive to a long-distance signal conduction. In this case, through the design of the knife-shaped structure and a conductive wire made of a special material (such as a flexible material, an elastic material, etc.), a special processing technique, and a special structure, the second electrode part 1920 may be transferred (e.g., the conductive wire may be electrically connected to the second electrode 1920), so that the electrode can implement a long-distance signal transmission.

In some embodiments, the electrodes can be designed to be non-slip. During the user's exercise, the electrode may move and/or fall off due to the pulling of the action, so the electrode needs to be fixed and anti-slip design. Referring to FIG. 19B, the second electrode part 1920 passes through the vicinity of the first plane, and the second electrode part 1920 can be fixed for the first time. Before the second electrode part 1920 performs signal transfer, the second electrode part 1920 may be fixed for the second time. It should be noted that the fixing method here may be physical connection such as bonding and welding, and the fixing position and fixing order may also be determined according to needs, which is not limited in this embodiment. In some embodiments, localized pressurization can be designed near the knife-shaped electrode structure. The design of local pressure near the knife-shaped electrode structure in the wearable apparatus can effectively enhance the adhesion force near the knife-shaped electrode structure to the human body, for example, pressurize the arm joints of the wearable apparatus. When the user is exercising, the arm is pulled. In this case, the pressurized arm joints can effectively reduce the displacement of the clothing base on the user's body, so that the sliding of the knife-shaped electrode structure arranged at the position of the human arm can be effectively avoided. In some embodiments, silica gel can be applied around the knife-shaped electrode structure to increase the friction between the knife-shaped electrode structure and the skin, thereby effectively avoiding the displacement of the knife-shaped electrode structure caused by the pulling of the user's movement. In some embodiments, the elastic design can be carried out near the knife-shaped electrode structure. The detailed description of the elastic design can be found in FIG. 17A and FIG. 17B, and will not be repeated in this embodiment. In some embodiments, it is also possible to carry out a hollow design near the knife-shaped electrode structure. The detailed description of the hollow design can be found in FIG. 18, which will not be repeated in this embodiment. In some embodiments, the adsorption design may further be performed near the knife-shaped electrode structure. The detailed description of the adsorption design can be found in FIG. 12 and FIG. 13, and will not be repeated in this embodiment.

It should be noted that the electrode modules in the above embodiments (e.g., the electrode module 100, the electrode module 200, the electrode module 300 and the EMG signal module in the wearable apparatus) can not only be applied to the collection of EMG signals, but also can release current stimulation Specific parts of the human body to achieve the corresponding reminder effect or massage relaxation effect. For example, when a user is exercising, the wearable apparatus may determine whether the user's fitness action is standardized based on body moving data, and may use the electrode module to release a current to remind the user of the exercise state.

It should be noted that different embodiments may have different beneficial effects. In different embodiments, the possible beneficial effects may be any one or combinations of the above, or any other possible beneficial effects.

Moreover, certain terminology has been used to describe embodiments of the present disclosure. For example, "one embodiment", "an embodiment", and/or "some embodiments" refer to a certain feature, structure or characteristic related to at least one embodiment of the present disclosure. Therefore, it should be emphasized and noted that two or more references to "an embodiment" or "one embodiment" or "an alternative embodiment" in different places in the present disclosure do not necessarily refer to the same embodiment. In addition, certain features, structures or characteristics of one or more embodiments of the present disclosure may be properly combined.

In addition, unless explicitly stated in the claims, the order of processing elements and sequences described in the disclosure, the use of numbers and letters, or the use of other designations are not used to limit the order of the process and methods of the disclosure. For example, although the system components described above may be implemented by hardware devices, they may also be implemented in a software-only solution, such as installing the described system on an existing server or a mobile device.

In the same way, it should be noted that in order to simplify the expression disclosed in the present disclosure and help the understanding of one or more embodiments thereof, in the foregoing description of the embodiments of the present disclosure, sometimes multiple features are combined into one embodiment, drawing or description thereof.

In some embodiments, numbers describing the quantity of components and attributes are used. It should be understood that such numbers used in the description of the embodiments use the modifiers "about", "approximately" or "substantially" in some examples. Unless otherwise stated, the "about", "approximately" or "substantially" indicates that the stated figure allows for a variation of $\pm 20\%$. Accordingly, in some embodiments, the numerical parameters used in the present disclosure and the claims are approximations that may vary depending upon the desired features of individual embodiments. In some embodiments, the numerical parameters should take into account the specified significant digits and use a general digit reservation method. Notwithstanding that the numerical fields and parameters used in some embodiments of the present disclosure to confirm the breadth of their ranges are approximations, in particular embodiments such numerical values are set as precisely as practicable.

**Claims**

1. A device for collecting and processing an electromyography (EMG) signal of a human body, comprising:

   an electrode module (100, 200, 300, 1200, 1300,1710) configured to collect the EMG signal of the human body, the electrode module (100, 200, 300, 1200, 1300,1710) including a base structure (103, 203, 303, 701, 1203, 1303), at least two electrodes, and a plurality of sucker structures (800, 1000, 1100, 1210, 1310), the at least two electrodes being arranged at intervals on a surface of the base structure (103, 203, 303, 701, 1203, 1303), the plurality of sucker structures (800, 1000, 1100, 1210, 1310) being arranged in an array or randomly distributed on surfaces of the at least two electrodes or on a surface of the base structure (103, 203, 303, 701, 1203, 1303),
   wherein each of the sucker structures (800, 1000, 1100, 1210, 1310) is a three-dimensional (3D) structure made of reversibly deformable material, the device being **characterised in that** each of the sucker structures (800, 1000, 1100, 1210, 1310) includes a hollow part (810, 1010) and an middle structure (1020, 1120) located in the hollow part (810, 1010) of the sucker structure (800, 1000, 1100, 1210, 1310), the hollow part (810, 1010) having an opening used to contact a human skin at an end of the sucker structure (800, 1000, 1100, 1210, 1310), a part of the middle structure (1020, 1120) being connected to a side wall of the sucker structure (800, 1000, 1100, 1210, 1310) where the hollow part (810, 1010) is located, other parts of the middle structure (1020, 1120) being fitted to, but not connected to the side wall of the sucker structure (800, 1000, 1100, 1210, 1310) where the hollow part (810, 1010) is located, and the middle structure (1020, 1120) dividing the hollow part (810, 1010) of the sucker structure (800, 1000, 1100, 1210, 1310) into a plurality of interconnected spatial areas.

2. The device of claim 1, wherein the at least two electrodes include a first electrode (101, 201, 301), a second electrode (102, 202, 302), a third electrode (204, 304), and a fourth electrode (305), the first electrode is arranged side by side with the third electrode, and the second electrode is arranged side by side with the fourth electrode, the first electrode (101, 201, 301) and the second electrode (102, 202, 302) being configured to be arranged along a length direction of a muscle fiber, the third electrode (204, 304) and the fourth electrode (305) being configured to be arranged along the length direction of the muscle fiber, the first electrode (101, 201, 301) and the third electrode (204, 304) being configured to be arranged along a width direction of the muscle fiber, the second electrode (102, 202, 302) and the fourth electrode (305) being configured to be arranged along the width direction of the muscle fiber; the first electrode (101, 201, 301) and the second electrode (102, 202, 302) being respectively connected to two input ends of a differential circuit to determine a first EMG signal, the third electrode (204, 304) and the fourth electrode (305) being respectively connected to two input ends of another differential circuit to determine a second EMG signal, and the first EMG signal and the second EMG signal being used to obtain EMG signal crosstalk effect information near the muscle fiber .

3. The device of claim 1, wherein the electrode module (100, 200, 300, 1200, 1300,1710) includes a plurality of protruding structures located on surfaces of the at least two electrodes or a surface of the base structure (103, 203, 303, 701, 1203, 1303).

4. The device of claim 3, wherein the protruding structures are hollow inside, and a hollow part (810, 1010) of each of the protruding structures has a filler.

5. The device of any of claims 1-4, wherein the electrode module (100, 200, 300, 1200, 1300,1710) further includes a plurality of bump structures located on the surface of the base structure (103, 203, 303, 701, 1203, 1303) or the surfaces of the at least two electrodes.

6. The device of claim 1, wherein the electrode module (100, 200, 300, 1200, 1300,1710) further includes a plurality of air holes located on the electrodes and/or the base structure (103, 203, 303, 701, 1203, 1303).

7. The device of claim 1, wherein each of the at least two electrodes includes a first electrode part (1910) and a second electrode part (1920), the first electrode part (1910) and the second electrode part (1920) being connected with each other, the first electrode part (1910) and the second electrode part (1920) having a height difference, wherein the first electrode part (1910) is configured to contact a human skin, and the second electrode part (1920) is configured to connect with a data wire so as to transmit an EMG signal collected by the first electrode part (1910).

8. A wearable apparatus, wherein the wearable apparatus includes an upper garment and pants, and the upper garment and the pants at least include a device for collecting an EMG signal of a human body according to any one of claims 1 to 7.

9. The wearable apparatus of claim 8, wherein the upper garment at least comprises:

at least one upper garment sensor module configured to collect upper body moving data;
at least one upper garment data processing module configured to receive and process the upper body moving data; and
an upper garment base configured to carry the at least one upper garment sensor module and the at least one upper garment data processing module.

10. The wearable apparatus of claim 9, wherein the at least one upper garment sensor module at least includes a first upper garment sensor module and a second upper garment sensor module, the first upper garment sensor module is located on a left side of the upper garment base, and the second upper garment sensor module is located on a right side of the upper garment base.

11. The wearable apparatus of any of claims 9-10, wherein the pants at least comprises:

at least one pants sensor module configured to collect lower body moving data;
at least one pants data processing module configured to receive and process the lower body moving data; and
a pants base configured to carry the at least one pants sensor module and the at least one pants data processing module.

12. The wearable apparatus of claim 11, wherein the at least one pants sensor module at least includes a first pants sensor module and a second pants sensor module, the first pants sensor module is located on a left side of the pants base, and the second pants sensor module is located on a right side of the pants base.

**Patentansprüche**

1. Vorrichtung zum Sammeln und Verarbeiten eines Elektromyographie-, (EMG-), Signals eines menschlichen Körpers, umfassend:

ein Elektrodenmodul (100, 200, 300, 1200, 1300, 1710), das konfiguriert ist, um das EMG-Signal des menschlichen Körpers zu sammeln, wobei das Elektrodenmodul (100, 200, 300, 1200, 1300, 1710) eine Basisstruktur (103, 203, 303, 701, 1203, 1303), mindestens zwei Elektroden und eine Vielzahl von Saugstrukturen (800, 1000, 1100, 1210, 1310) beinhaltet, wobei die mindestens zwei Elektroden in Intervallen auf einer Oberfläche der Basisstruktur (103, 203, 303, 701, 1203, 1303) angeordnet sind, wobei die Vielzahl von Saugstrukturen (800, 1000, 1100, 1210, 1310) in einem Array oder zufällig verteilt auf Oberflächen der mindestens zwei Elektroden oder auf einer Oberfläche der Basisstruktur (103, 203, 303, 701, 1203, 1303) angeordnet sind,
wobei jede der Saugerstrukturen (800, 1000, 1100, 1210, 1310) eine dreidimensionale (3D) Struktur ist, die aus reversibel verformbarem Material gefertigt ist, wobei die Vorrichtung **dadurch gekennzeichnet ist, dass** jede der Saugerstrukturen (800, 1000, 1100, 1210, 1310) einen hohlen Teil (810, 1010) und eine mittlere Struktur (1020, 1120) beinhaltet, die sich im hohlen Teil (810, 1010) der Saugerstruktur (800, 1000, 1100, 1210, 1310) befindet, wobei der hohle Teil (810, 1010) eine Öffnung aufweist, die verwendet wird, um Kontakt mit einer menschlichen Haut an einem Ende der Saugerstruktur (800, 1000, 1100, 1210, 1310) aufzunehmen, wobei ein Teil der mittleren Struktur (1020, 1120) mit einer Seitenwand der Saugerstruktur (800, 1000, 1100, 1210, 1310) verbunden ist, wo sich der hohle Teil (810, 1010) befindet, wobei andere Teile der mittleren Struktur (1020, 1120) an der Seitenwand der Saugerstruktur (800, 1000, 1100, 1210, 1310), wo sich der hohle Teil (810, 1010) befindet, angebracht, aber nicht mit dieser verbunden sind,
und die mittlere Struktur (1020, 1120) den hohlen Teil (810, 1010) der Saugerstruktur (800, 1000, 1100, 1210, 1310) in eine Vielzahl miteinander verbundener räumlicher Bereiche unterteilt.

2. Vorrichtung nach Anspruch 1, wobei die mindestens zwei Elektroden eine erste Elektrode (101, 201, 301), eine zweite Elektrode (102, 202, 302), eine dritte Elektrode (204, 304) und eine vierte Elektrode (305) beinhalten, wobei die erste Elektrode Seite an Seite mit der dritten Elektrode angeordnet ist, und die zweite Elektrode Seite an Seite mit der vierten Elektrode angeordnet ist, wobei die erste Elektrode (101, 201, 301) und die zweite Elektrode (102, 202, 302) konfiguriert sind, um entlang einer Längsrichtung einer Muskelfaser angeordnet zu sein, wobei die dritte Elektrode (204, 304) und die vierte Elektrode (305) konfiguriert sind, um entlang der Längsrichtung der Muskelfaser angeordnet zu sein, wobei die erste Elektrode (101, 201, 301) und die dritte Elektrode (204, 304) konfiguriert sind, um entlang einer Breitenrichtung der Muskelfaser angeordnet zu sein, wobei die zweite Elektrode (102, 202, 302) und die vierte Elektrode (305) konfiguriert sind, um entlang der Breitenrichtung der Muskelfaser angeordnet zu sein; die erste Elektrode (101,201,301) und die zweite Elektrode (102, 202, 302) jeweils mit zwei Eingangsenden einer Differenzialschaltung verbunden sind, um ein erstes EMG-Signal zu bestimmen, die dritte Elektrode (204, 304) und die vierte Elektrode (305) jeweils mit zwei Eingangsenden einer anderen Differenzialschaltung verbunden sind, um ein zweites EMG-Signal zu bestimmen, und das erste EMG-Signal und das zweite EMG-Signal verwendet werden, um Informationen zum EMG-Signal-Übersprecheffekt in der Nähe der Muskelfaser zu erhalten.

3. Vorrichtung nach Anspruch 1, wobei das Elektrodenmodul (100, 200, 300, 1200, 1300, 1710) eine Vielzahl von hervorstehenden Strukturen beinhaltet, die sich auf Oberflächen der mindestens zwei Elektroden oder einer Oberfläche der Basisstruktur (103, 203, 303, 701, 1203, 1303) befinden.

4. Vorrichtung nach Anspruch 3, wobei die hervorstehenden Strukturen innen hohl sind und ein hohler Teil (810, 1010) jeder der hervorstehenden Strukturen einen Füllstoff aufweist.

5. Vorrichtung nach einem der Ansprüche 1-4, wobei das Elektrodenmodul (100, 200, 300, 1200, 1300, 1710) weiter eine Vielzahl von Erhebungsstrukturen beinhaltet, die sich auf der Oberfläche der Basisstruktur (103, 203, 303, 701, 1203, 1303) oder den Oberflächen der mindestens zwei Elektroden befinden.

6. Vorrichtung nach Anspruch 1, wobei das Elektrodenmodul (100, 200, 300, 1200, 1300, 1710) weiter eine Vielzahl von Luftlöchern beinhaltet, die sich an den Elektroden und/oder der Basisstruktur (103, 203, 303, 701, 1203, 1303) befinden.

7. Vorrichtung nach Anspruch 1, wobei jede der mindestens zwei Elektroden einen ersten Elektrodenteil (1910) und einen zweiten Elektrodenteil (1920) beinhaltet, wobei der erste Elektrodenteil (1910) und der zweite Elektrodenteil (1920) miteinander verbunden sind, und der erste Elektrodenteil (1910) und der zweite Elektrodenteil (1920) einen Höhenunterschied aufweisen, wobei der erste Elektrodenteil (1910) konfiguriert ist, um mit einer menschlichen Haut in Kontakt zu treten, und der zweite Elektrodenteil (1920) konfiguriert ist, um mit einem Datenkabel verbunden zu werden, um ein vom ersten Elektrodenteil (1910) gesammeltes EMG-Signal zu übertragen.

8. Tragbare Einrichtung, wobei die tragbare Einrichtung ein Oberbekleidungsstück und eine Hose beinhaltet und das Oberbekleidungsstück und die Hose mindestens eine Vorrichtung zum Sammeln eines EMG-Signals eines menschlichen Körpers nach einem der Ansprüche 1 bis 7 beinhalten.

9. Tragbare Einrichtung nach Anspruch 8, wobei das Oberbekleidungsstück mindestens umfasst:

mindestens ein Sensormodul der Oberbekleidung, das konfiguriert ist, um Daten über die Bewegungen des Oberkörpers zu sammeln; mindestens ein Datenverarbeitungsmodul der Oberbekleidung, das konfiguriert ist, um Daten zur Bewegung des Oberkörpers zu empfangen und zu verarbeiten; und eine Oberbekleidungsbasis, die konfiguriert ist, um das mindestens eine Sensormodul der Oberbekleidung und das mindestens eine Datenverarbeitungsmodul der Oberbekleidung zu tragen.

10. Tragbare Einrichtung nach Anspruch 9, wobei das mindestens eine Sensormodul der Oberbekleidung mindestens ein erstes Sensormodul der Oberbekleidung und ein zweites Sensormodul der Oberbekleidung beinhaltet, sich das erste Sensormodul der Oberbekleidung auf einer linken Seite der Oberbekleidungsbasis befindet, und sich das zweite Sensormodul der Oberbekleidung auf einer rechten Seite der Oberbekleidungsbasis befindet.

11. Tragbare Einrichtung nach einem der Ansprüche 9-10, wobei die Hose mindestens umfasst:

mindestens ein Sensormodul der Hose, das konfiguriert ist, um Daten über die Bewegungen des Unterkörpers zu sammeln; mindestens ein Datenverarbeitungsmodul der Hose, das konfiguriert ist, um Daten zur Bewegung des Unterkörpers zu empfangen und zu verarbeiten; und eine Hosenbasis, die konfiguriert ist, um das mindestens eine Sensormodul der Hose und das mindestens eine Datenverarbeitungsmodul der Hose zu tragen.

12. Tragbare Einrichtung nach Anspruch 11, wobei das mindestens eine Sensormodul der Hose mindestens ein erstes Sensormodul der Hose und ein zweites Sensormodul der Hose beinhaltet, sich das erste Sensormodul der Hose auf einer linken Seite der Hosenbasis befindet, und sich das zweite Sensormodul der Hose auf einer rechten Seite der Hosenbasis befindet.

**Revendications**

1. Dispositif de collecte et de traitement d'un signal d'électromyographie (EMG) d'un corps humain, comprenant :

   un module d'électrodes (100, 200, 300, 1200, 1300, 1710) configuré pour collecter le signal EMG du corps humain, le module d'électrodes (100, 200, 300, 1200, 1300, 1710) incluant une structure de base (103, 203, 303, 701, 1203, 1303), au moins deux électrodes et une pluralité de structures à ventouse (800, 1000, 1100, 1210, 1310), les au moins deux électrodes étant agencées à des intervalles sur une surface de la structure de base (103, 203, 303, 701, 1203, 1303), la pluralité de structures à ventouse (800, 1000, 1100, 1210, 1310) étant agencée en une matrice ou répartie aléatoirement sur des surfaces des au moins deux électrodes ou sur une surface de la structure de base (103, 203, 303, 701, 1203, 1303),
   dans lequel chacune des structures à ventouse (800, 1000, 1100, 1210, 1310) est une structure tridimensionnelle (3D) constituée d'un matériau déformable de manière réversible, le dispositif étant **caractérisé en ce que** chacune des structures à ventouse (800, 1000, 1100, 1210, 1310) inclut une partie creuse (810, 1010) et une structure centrale (1020, 1120) située dans la partie creuse (810, 1010) de la structure à ventouse (800, 1000, 1100, 1210, 1310), la partie creuse (810, 1010) présentant une ouverture utilisée pour entrer en contact avec une peau humaine à une extrémité de la structure à ventouse (800, 1000, 1100, 1210, 1310), une partie de la structure centrale (1020, 1120) étant reliée à une paroi latérale de la structure à ventouse (800, 1000, 1100, 1210, 1310) où se trouve la partie creuse (810, 1010), d'autres parties de la structure centrale (1020, 1120) étant montées sur, mais non reliées à, la paroi latérale de la structure à ventouse (800, 1000, 1100, 1210, 1310) où se trouve la partie creuse (810, 1010), et la structure centrale (1020, 1120) divisant la partie creuse (810, 1010) de la structure à ventouse (800, 1000, 1100, 1210, 1310) en une pluralité de zones spatiales reliées entre elles.

2. Dispositif selon la revendication 1, dans lequel les au moins deux électrodes incluent une première électrode (101, 201, 301), une deuxième électrode (102, 202, 302), une troisième électrode (204, 304) et une quatrième électrode (305), la première électrode étant agencée côte à côte avec la troisième électrode et la deuxième électrode étant agencée côte à côte avec la quatrième électrode, la première électrode (101, 201, 301) et la deuxième électrode (102, 202, 302) étant configurées pour être agencées dans le sens de la longueur d'une fibre musculaire, la troisième électrode (204, 304) et la quatrième électrode (305) étant configurées pour être agencées dans le sens de la longueur de la fibre musculaire, la première électrode (101, 201, 301) et la troisième électrode (204, 304) étant configurées pour être agencées dans le sens de la largeur de la fibre musculaire, la deuxième électrode (102, 202, 302) et la quatrième électrode (305) étant configurées de façon à être agencées dans le sens de la largeur de la fibre musculaire ; la première électrode (101, 201, 301) et la deuxième électrode (102, 202, 302) étant respectivement reliées à deux extrémités d'entrée d'un circuit différentiel pour déterminer un premier signal EMG, la troisième électrode (204, 304) et la quatrième électrode (305) étant respectivement reliées à deux extrémités d'entrée d'un autre circuit différentiel pour déterminer un second signal EMG, et le premier signal EMG et le second signal EMG étant utilisés pour obtenir des informations sur un effet de diaphonie des signaux EMG à proximité de la fibre musculaire.

3. Dispositif selon la revendication 1, dans lequel le module d'électrodes (100, 200, 300, 1200, 1300, 1710) inclut une pluralité de structures saillantes situées sur des surfaces des au moins deux électrodes ou sur une surface de la structure de base (103, 203, 303, 701, 1203, 1303).

4. Dispositif selon la revendication 3, dans lequel les structures saillantes sont creuses à l'intérieur et une partie creuse (810, 1010) de chacune des structures saillante présente une charge.

5. Dispositif selon l'une quelconque des revendications 1-4, dans lequel le module d'électrodes (100, 200, 300, 1200, 1300, 1710) inclut en outre une pluralité de structures à protubérance situées sur la surface de la structure de base (103, 203, 303, 701, 1203, 1303) ou sur les surfaces des au moins deux électrodes.

6. Dispositif selon la revendication 1, dans lequel le module d'électrodes (100, 200, 300, 1200, 1300, 1710) inclut en outre une pluralité de trous d'aération situés sur les électrodes et/ou sur la structure de base (103, 203, 303, 701, 1203, 1303).

7. Dispositif selon la revendication 1, dans lequel chacune des au moins deux électrodes inclut une première partie d'électrode (1910) et une seconde partie d'électrode (1920), la première partie d'électrode (1910) et la seconde partie d'électrode (1920) étant reliées l'une à l'autre, la première partie d'électrode (1910) et la seconde partie d'électrode (1920) présentant une différence de hauteur, dans lequel la première partie d'électrode (1910) est configurée pour entrer en contact avec une peau humaine et la seconde partie d'électrode (1920) est configurée pour se brancher à un fil de données de manière à transmettre un signal EMG collecté par la première partie d'électrode (1910).

8. Appareil à porter sur soi, dans lequel l'appareil à porter sur soi inclut un vêtement pour le haut du corps et un pantalon, et le vêtement pour le haut du corps et le pantalon incluent au moins un dispositif de collecte d'un signal EMG d'un corps humain selon l'une quelconque des revendications 1 à 7.

9. Appareil à porter sur soi selon la revendication 8, dans lequel le vêtement pour le haut du corps comprend au moins :

   au moins un module de capteurs de vêtement pour le haut du corps configuré pour collecter des données de mouvements du haut du corps ;
   au moins un module de traitement de données de vêtement pour le haut du corps configuré pour recevoir et traiter les données de mouvements du haut du corps ; et
   une base de vêtement pour le haut du corps configurée pour porter le au moins un module de capteurs de vêtement pour le haut du corps et le au moins un module de traitement des données de vêtement pour le haut du corps.

10. Appareil à porter sur soi selon la revendication 9, dans lequel le au moins un module de capteurs de vêtement pour le haut du corps inclut au moins un premier module de capteurs de vêtement pour le haut du corps et un second module de capteurs de vêtement pour le haut du corps, le premier module de capteurs de vêtement pour le haut du corps est situé sur un côté gauche de la base de vêtement pour le haut du corps et le second module de capteurs de vêtement pour le haut du corps est situé sur un côté droit de la base de vêtement pour le haut du corps.

11. Appareil à porter sur soi selon l'une quelconque des revendications 9-10, dans lequel le pantalon comprend au moins :

   au moins un module de capteurs du pantalon configuré pour collecter des données de mouvements du bas du corps ;
   au moins un module de traitement de données de pantalon configuré pour recevoir et traiter les données de mouvements du bas du corps ; et
   une base de pantalon configurée pour porter le au moins un module de capteurs de pantalon et le au moins un module de traitement de données de pantalon.

12. Appareil à porter sur soi selon la revendication 11, dans lequel le au moins un module de capteurs de pantalon inclut au moins un premier module de capteurs de pantalon et un second module de capteurs de pantalon, le premier module de capteurs de pantalon est situé sur un côté gauche de la base de pantalon et le second module de capteurs de pantalon est situé sur un côté droit de la base de pantalon.

100    101         102    103

FIG. 1

FIG. 2

FIG. 3

FIG. 4

Measurement point

Electrode

**R2**  **R2**

r1
r1
r1

**R1**  **R1**

skin

**R4**  **R4**  **R4**

**R5**  **R5**

**EMG**

FIG. 5

FIG. 6

701

720

710

FIG. 7A

Measurement point

electrode

R1    R1    R1

R2    R2

skin

R4

EMG

FIG. 7B

800   810

811

skin

FIG. 8

FIG. 9

FIG. 10

FIG. 11

FIG. 12

FIG. 13

FIG. 14

1500

FIG. 15

FIG. 16A

FIG. 16B

1720

1710

FIG. 17A

1740

1730

FIG. 17B

<u>1800</u>

FIG. 18

1910

1920

FIG. 19A

First plane

1920

1910

Second plane

FIG. 19B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014343392 A1

- WO 2020254833 A1